# EUROPÄISCHE PATENTSCHRIFT

(11) **EP 2 065 822 B1**
(45) Veröffentlichungstag und Bekanntmachung des Hinweises auf die Patenterteilung: **13.07.2016**
(21) Anmeldenummer: 08018209.0
(22) Anmeldetag: 17.10.2008
(51) Int. Cl.: G06F 19/00

(54) **Verfahren und System zum Umpacken von Arnzeimitteln**
Method and system for repacking medicines
Procédé et système destinés au reconditionnement de médicaments

(30) Priorität: 29.11.2007 DE 102007057881
(43) Veröffentlichungstag der Anmeldung: 03.06.2009
(73) Patentinhaber: Deutsche Blistergesellschaft mbH, 76532 Baden-Baden (DE)
(72) Erfinder: Holdermann, Hans-Werner, 76532 Baden-Baden (DE)
(74) Vertreter: Lemcke, Brommer & Partner Patentanwälte Partnerschaft mbB

(56) Entgegenhaltungen:
- WO-A1-2005/054059
- WO-A1-2005/087175
- WO-A1-2006/055515
- WO-A2-2004/036481
- US-A- 5 208 762
- US-A- 5 468 110
- US-A1- 2007 043 469

## Beschreibung

Die vorliegende Erfindung betrifft ein Verfahren zum Umpacken von Arzneimitteln, ein Arzneimittelumpacksystem, insbesondere zum Durchführen des erfindungsgemäßen Verfahrens, sowie zur Durchführung des erfindungsgemäßen Verfahrens ansetzbare Computerprogrammprodukte.

Zumindest in Deutschland werden Medikamente oder Arzneimittel ausschließlich in arzneimittelrechtlich zulässigen bzw. zugelassenen Verpackungen vertrieben, insbesondere in Blisterverpackungen. Eine Abgabe von Arzneimitteln, die in einer Verpackung regelmäßig eine Mehrzahl von Arzneimitteleinheiten, wie Tabletten, Kapseln oder dergleichen, enthalten, in anderer Form ist nicht zulässig.

Insbesondere in Pflegeheimen, Krankenhäusern oder vergleichbaren Einrichtungen, in denen eine Vielzahl von Patienten mit bestimmten, verordneten Arzneimitteln zu vorgegebenen Zeiten versorgt werden müssen, fällt beim Entnehmen der Arzneimitteleinheiten aus ihren Verpackungen und der anschließenden patientenindividuellen Zuteilung ein hohes Maß an Arbeit an, mit der in größeren Anstalten bestimmte Fachkräfte ausschließlich beschäftigt sein können.

So ist nach dem Stand der Technik bekannt, dass beispielsweise in Pflegeheimen oder Krankenhäusern Pflegekräfte damit beschäftigt sind, Arzneimitteleinheiten aus ihren Verpackungen zu entnehmen, beispielsweise zu entblistern, und die Arzneimitteleinheiten gemäß einer ärztlichen Verordnung für einen bestimmten Patienten, was vorliegend auch als Patientenmedikationsdaten oder patientenspezifische Medikationsdaten bezeichnet wird, in hierfür vorgesehene Behältnisse umzupacken. Zu diesem Zweck ist insbesondere die Verwendung von mehrfach unterteilten Behältnissen bekannt, bei denen in einzelnen Fächern jeweils die von einem bestimmten Patienten zu einem bestimmten Zeitpunkt (Wochentag, Ührzeit, ...) einzunehmenden Arzneimitteleinheiten enthalten sind.

Hierbei ist insbesondere als nachteilig anzusehen, dass ein derartiges Verfahren aufgrund des hohen Personalaufwands einen entsprechend hohen Kostenaufwand verursacht, wobei zudem aufgrund der relativ monotonen Tätigkeit insbesondere in großen Anstalten die Gefahr einer folgenschweren Fehlmedikation besteht, wenn Arzneimitteleinheiten falsch zugeteilt werden. Zudem ist eine Dokumentation der erfolgenden Arbeitsabläufe zur Fehlerfindung oder -vermeidung nicht möglich, so dass eine gewerbemäßige Anwendung der bekannten Verfahren ausscheidet.

Aus der US 2007/0043469 A1 ist ein Arzneimittelangabesystem bekannt bei dem Produktbehälter mit Arzneimitteleinheiten aus einem Transport karton entnommen und in eine automatisierte Regalanordnung ein sortiert werden, woraus die an bezugsberechtigte Personen ausgegeben werden können.

Aus der US 5,468,110 ist ein automatisiertes System zum Auswählen von Packungen aus einem Lagerbereich bekannt, welches die Überprüfung eines Arbeitsschritts, beispielsweise der Auswahl eines Arzneimittels, gegen einen Auftrag umfasst.

Die US 5,208,762 offenbart ein automatisiertes Abfüllsystem auf der Basis von Rezeptdaten.

Der Erfindung liegt die Aufgabe zugrunde, ein Verfahren und ein System zum Umpacken von Arzneimitteln anzugeben, welche sich durch verbesserte Kosteneffizienz, erhöhte Fehlersicherheit und gegebene Dokumentationsfähigkeit auszeichnen und die somit auch für industrielle oder gewerbliche Arzneimittelumpackprozesse verwendbar sind.

Die Erfindung löst die Aufgabe mittels eines Verfahrens zum Umpacken von Arzneimitteln mit den Merkmalen des Patentanspruchs 1 und mittels eines Arzneimittelumpacksystems mit den Merkmalen des Patentanspruchs 9. Weitere Aspekte der vorliegenden Erfindung betreffen Computerprogrammprodukte zum Ausführen des erfindungsgemäßen Verfahrens bzw. zum Betreiben des erfindungsgemäßen Arzneimittelumpacksystems.

Ein erstes Beispiel umfasst ein Verfahren in Form einer jeweiligen einteldosierten Artneiform zum Umpacken von Arzneimitteln, wobei eine Anzahl von Arzneimitteleinheiten eines entsprechenden Arzneimittels aus einer betreffenden ersten Verpackung entnommen und ein zugehöriger arzneimittelspezifischer Datensatz erfasst wird und wobei wenigstens eine Teilmenge der entnommenen Arzneimitteleinheiten an einen Dosier- und Verpackungsautomaten übergeben wird, der wenigstens eine Arzneimitteleinheit aus einer Anzahl vorgegebener Arzneimittel entsprechend einem patientenspezifischen Medikationsdatensatz ausgibt und in einer patientenspezifischen Verpackung einpackt und dabei verpackungsspezifische Ausgabedaten erzeugt, welche den in dem Dosier- und Verpackungsautomaten ablaufenden Ausgabe- und Verpackungsvorgang dokumentieren, worauf ein automatischer Abgleichs der verpackungsspezifischen Ausgabedaten des Dosier- und Verpackungsautomaten und der patientenspezifischen Verpackung, insbesondere deren Inhalts, mit dem patientenspezifischen Medikationsdatensatz vorgenommen und bei Nichtkonsistenz ein entsprechendes Steuersignal ausgelöst wird.

Ein weiteres Beispiel umfasst ein Arzneimittelumpacksystem, insbesondere zum Durchführen des erfindungsgemäßen Verfahrens, aufweisend:
- eine Entnahme- und Erfassungsstation, die zum Entnehmen einer Anzahl von Arzneimitteleinheiten in Form jeweils einteldosierter Arzneiformen eines entsprechenden Arzneimittels aus einer betreffenden ersten Verpackung und zum Erfassen eines zugehörigen arzneimittelspezifischen Datensatzes in vorzugsweise elektronischer Form ausgebildet ist;
- optional eine Bevorratungsstation zum Lagern der entnommenen Arzneimitteleinheiten getrennt nach Arzneimitteln;
- eine Abfüll- und Verpackungsstation mit einem Dosier- und Verpackungsautomaten, die zum Ausgeben wenigstens einer Arzneimitteleinheit aus einer Anzahl vorgegebener Arzneimittel entsprechend einem patientenspezifischen Medikationsdatensatz und zum Einpacken derselben in eine patientenspezifische Verpackung ausgebildet ist;
- eine Abgleich- und Prüfstation, die zum Überprüfen der patientenspezifischen Verpackung, insbesondere deren Inhalts, ausgebildet ist,
wobei die Abfüll- und Verpackungsstation zum Veranlassen eines ersten Abgleichs von verpackungsspezifischen Ausgabedaten, die von dem Dosier- und Verpackungsautomaten beim Ausgeben und Verpacken erzeugt werden, mit dem patientenspezifischen Medikationsdatensatz und zum Veranlassen des Ausgebens eines entsprechenden Steuersignals bei Nichtkonsistenz ausgebildet ist und wobei die Abgleich- und Prüfstation zum Veranlassen eines zweiten Abgleichens der patientenspezifischen Verpackung, insbesondere deren Inhalts, mit dem patientenspezifischen Medikationsdatensatz und zum Veranlassen des Auslösens eines entsprechenden Steuersignals bei Nichtkonsistenz ausgebildet ist, wobei die Entnahme- und Erfassungsstation mit der Abfüll- und Verpackungsstation und mit der Abgleich- und Prüfstation daten- und steuerungstechnisch verbunden ist, um den ersten und den zweiten Abgleich vorzunehmen.

Ein weiteres Beispiel umfasst ein Computerprogrammprodukt mit auf einem Datenträger gespeicherten Programmanweisungen, die zur Ausführung durch eine Ablaufsteuerungseinheit des erfindungsgemäßen Arzneimittelumpacksystems vorgesehen sind, um elektronische Datensätze und/oder Steuersignale, die bei Ablauf des erfindungsgemäßen Verfahrens auftreten, zu erzeugen, zu archivieren und/oder zu visualisieren.

Schließlich schafft die vorliegende Erfindung gemäß einem weiteren Beispiel ein Computerprogrammprodukt mit auf einem Datenträger gespeicherten Programmanweisungen, bei deren Ausführung auf einem geeigneten Rechnersystem eine Oberfläche, insbesondere eine grafische Benutzeroberfläche (graphical user interface - GUI), zum Erzeugen von patientenspezifischen Medikationsdaten oder einem Patientenmedikationsdatensatz in Relation mit dem erfindungsgemäßen Verfahren und/oder dem erfindungsgemäßen Arzneimittelumpacksystem erzeugt wird.

Weitere Beispiele sind jeweils in entsprechenden Unteransprüchen angegeben, deren Wortlaut hiermit durch ausdrückliche Bezugnahme zum Gegenstand der Beschreibung gemacht wird, um unnötige Textwiederholungen zu vermeiden.

Im Zuge des erfindungsgemäßen Verfahrens zum Umpacken von Arzneimitteln ist also vorgesehen, dass eine Anzahl von Arzneimitteleinheiten, wie Tabletten, Kapseln oder dergleichen, eines entsprechenden Arzneimittels aus einer betreffenden ersten Verpackung entnommen wird, beispielsweise aus einer Blisterpackung durch Entblistern. Alternativ kann auch vorgesehen sein, dass die erste Verpackung eine Blisterpackung umfasst, und dass das Entnehmen durch Trennen zwischen einzelnen Blistern ohne Öffnen derselben erfolgt. Dabei wird - vorzugsweise automatisch - ein zugehöriger arzneimittelspezifscher Datensatz in vorzugsweise elektronischer Form erfasst. Dies kann beispielsweise durch Einlesen eines auf der ersten Verpackung oder einer zugehörigen Arzneimittelumverpackung angegebenen Strich- oder Barcodes erfolgen. Dann wird wenigstens eine Teilmenge der entnommenen Arzeimitteleinheiten an einen Dosier- und Verpackungsautomaten übergeben. Im Zuge einer Weiterbildung des erfindungsgemäßen Verfahrens kann allerdings vorgesehen sein, dass zumindest einige der entnommenen Arzneimitteleinheiten zunächst in einer optionalen Bevorratungsstation geeignet zwischengelagert und erst bei Bedarf an den Dosier- und Verpackungsautomaten übergeben werden.

Im weiteren Verlauf des erfindungsgemäßen Verfahrens gibt dann der Dosierund Verpackungsautomat entsprechend einem patientenspezifischen Medikationsdatensatz oder Patientenmedikationsdaten wenigstens eine Arzneimitteleinheit aus einer Anzahl vorgegebener Arzneimittel aus und packt diese in einer zweiten Verpackung ein, die im Rahmen der vorliegenden Anmeldung als patientenspezifische Verpackung bezeichnet wird. Dabei erzeugt der Dosierund Verpackungsautomat entsprechende verpackungsspezifische Ausgabedaten, die den in dem Automaten ablaufenden bzw. abgelaufenen Ausgabe- und Verpackungsvorgang protokollieren oder dokumentieren. Die patientenspezifische Verpackung fasst demgemäß gegebenenfalls mehrere, auch verschiedene Arzneimitteleinheiten gemäß den Patientenmedikationsdaten zu einer (Verpackungs-)Einheit zusammen. Insbesondere kann eine patientenspezifische Verpackung alle diejenigen Arzneimitteleinheiten enthalten, die ein bestimmter Patient zu einer gegebenen Zeit einnehmen soll.

Anschließend erfolgt im Zuge des erfindungsgemäßen Verfahrens ein automatischer Abgleich der verpackungsspezifischen Ausgabedaten und/oder der patientenspezifischen Verpackung, insbesondere deren Inhalts, mit dem patientenspezifischen Medikationsdatensatz. Mit anderen Worten, die hergestellte patientenspezifische Verpackung und insbesondere deren Inhalt wird einfach oder doppelt bzw. redundant mit dem patientenspezifischen Medikationsdatensatz verglichen, und bei Nichtkonsistenz wird jeweils ein entsprechendes Steuersignal ausgelöst. Der automatische Abgleich bzw. die automatischen Abgleiche gewährleistet bzw. gewährleisten also, dass eine gegebene patientenspezifische Verpackung mit dem zugrunde liegenden patientenspezifischen Medikationsdatensatz übereinstimmt bzw. dessen Medikationsinhalt korrekt wiedergibt.

Die erwähnte Nichtkonsistenz kann sich insbesondere dadurch ergeben, dass eine patientenspezifische Verpackung eine Anzahl von Arzneimitteleinheiten oder Arten von Arzneimitteleinheiten enthält, die nicht mit denjenigen Arzneimitteleinheiten bzw. deren Anzahl übereinstimmt, welche der patientenspezifische Medikationsdatensatz vorschreibt, z. B. wenn der Dosier- und Verpackungsautomat eine Fehlfunktion aufweist oder wenn fälschlicherweise Arzneimitteleinheiten gemäß Auftragsdaten in Form bereits gelöschter oder stornierter Patientenmedikationsdaten verpackt wurden. Bei den genannten Steuersignalen kann es sich insbesondere um Fehlersignale handeln, welche das erfindungsgemäße Verfahren unterbrechen und/oder das Eingreifen einer Bedienperson veranlassen. Zusätzlich oder alternativ können die genannten Steuersignale auch lediglich archiviert und/oder visualisiert werden, um nachträglich fehlerhafte patientenspezifische Verpackungen auffinden und aussondern zu können.

Im Rahmen einer bevorzugten Weiterbildung des erfindungsgemäßen Verfahrens enthält der arzneimittelspezifische Datensatz, der im Zusammenhang mit der Entnahme der Arzneimitteleinheiten aus der ersten Verpackung generiert wird, wenigstens eine der folgenden Angaben:
- die Art des Arzneimittels, insbesondere seine Pharmazentralnummer, PZN;
- die Anzahl der entnommenen und/oder fehlerhaften Arzneimitteleinheiten der Verpackung;
- Haltbarkeitsangaben, wie ein Verfallsdatum oder dergleichen; und
- den Aktualisierungsstand (Aktualisierungsdatum) der Packungsbeilage bzw. Produktinformationen.

Somit steht für jedes im Verfahren befindliche Arzneimittel, insbesondere sogar für jede einzelne entnommene Arzneimitteleinheit, in Weiterbildung der Erfindung ein umfangreicher Datensatz zur Verfügung, der alle arzneimittelrechtlich relevanten Angaben bezüglich des Arzneimittels bzw. der Arzneimitteleinheiten enthält.

In Weiterbildung des erfindungsgemäßen Verfahrens ist vorgesehen, dass jedem arzneimittelspezifischen Datensatz automatisch noch eine so genannte Verwendbarkeitszeit in Abhängigkeit von der Entnahmezeit zugewiesen wird. Dabei ist die Entnahmezeit derjenige Zeitpunkt, in dem die Arzneimitteleinheiten aus der ersten Verpackung entnommen werden bzw. in dem die Entnahme datentechnisch erfasst wird. Die zugewiesene Verwendbarkeitszeit orientiert sich vorteilhafterweise an einer bekannten oder angenommenen Stabilitätszeit für die entnommenen Arzneimitteleinheiten, wodurch berücksichtigt wird, dass eine entnommene Arzneimitteleinheit unter Umständen aufgrund von Umgebungseinflüssen (Feuchtigkeit oder dergleichen) ihre Wirksamkeit zumindest teilweise verlieren oder ihre Beschaffenheit ändern kann. Im Rahmen der genannten Weiterbildung des erfindungsgemäßen Verfahrens ist also eine entnommene Arzneimitteleinheit nur während einer gewissen vorgegebenen Verwendbarkeitszeit weiter verarbeitbar, insbesondere ausgebbar. Typischerweise kann die Verwendbarkeitszeit bei 3 oder 6 Monaten liegen. Insbesondere kann auch für jedes Arzneimittel bzw. jeden Typ von Arzneimitteleinheit eine eigene Verwendbarkeitszeit vorgegeben sein.

Gemäß einer anderen Weiterbildung des erfindungsgemäßen Verfahrens wird dieses durch eine automatische, insbesondere rechnergestützte Ablaufsteuerungseinheit gesteuert. Diese Steuerungseinheit, die zentral oder zumindest teilweise dezentral ausgebildet sein kann, greift bei der Ansteuerung des Dosier- und Verpackungsautomaten oder sogar bereits bei Eingang zu verarbeitender Patientenmedikationsdaten (Auftragsdaten; Auftragseingang) auf den arzneimittelspezifischen Datensatz zu, so dass das Ausgeben von Arzneimitteleinheiten in Abhängigkeit von den Daten erfolgt, die in dem arzneimittelspezifischen Datensatz enthalten sind. Dies betrifft insbesondere die bereits erwähnten Haltbarkeitsangaben und/oder die oben definierte Verwendbarkeitszeit. Auf diese Weise kann sichergestellt werden, dass der Dosier- und Verpackungsautomat keine Arzneimitteleinheiten ausgibt, deren Haltbarkeitsdatum bereits überschritten oder deren Verwendbarkeitszeit bereits abgelaufen ist. Eine andere Weiterbildung des erfindungsgemäßen Verfahrens sieht vor, dass beim Ansteuern zumindest des Dosier- und Verpackungsautomaten zusätzlich noch auf die patientenspezifischen Medikationsdaten zugegriffen wird und dass das Ausgeben von Arzneimitteleinheiten in Abhängigkeit von einem Vergleich der in den genannten Datensätzen enthaltenen Daten erfolgt. Auf diese Weise lässt sich insbesondere ausschließen, dass in einer patientenspezifischen Verpackung Arzneimitteleinheiten eingepackt werden, deren Haltbarkeitsdatum und/oder Verwendbarkeitszeit vor dem vorgeschriebenen Einnahmezeitpunkt liegt bzw. endet.

In der Praxis existieren Arzneimittel, die sich nicht in der Art oder Zusammensetzung der einzelnen Arzneimitteleinheiten sondern lediglich im Hinblick auf eine Packungsgröße (N1, N2, ...) der ersten Verpackung unterscheiden. Solchen Arzneimitteln sind unterschiedliche Pharmazentralnummern zugeordnet, welche beim Erstellen des arzneimittelspezifischen Datensatzes im Zuge des erfindungsgemäßen Verfahrens regelmäßig mit erfasst werden. Im Zuge einer Weiterbildung des erfindungsgemäßen Verfahrens ist es allerdings möglich, dass derartigen Arzneimitteln beim Erfassen des arzneimittelspezifischen Datensatzes automatisch noch eine übergeordnete, gemeinsame Kennzeichnung zugewiesen wird, beispielsweise eine geeignete Nummer, anhand der sich die entsprechenden Arzneimitteleinheiten als das gleiche Arzneimittel identifizieren lassen, was zusätzliche Flexibilität beim Ausgeben und/oder Verpacken der Arzneimitteleinheiten und/oder beim anschließenden Abgleichen schafft.

Der Patientenmedikationsdatensatz kann in Weiterbildung des erfindungsgemäßen Verfahrens wenigstens die folgenden Daten enthalten:
- Identifikationsdaten des Patienten, insbesondere Namen, Geschlecht, Alter, Wohnort, Behandlungsort oder dergleichen;
- Medikationsdaten, insbesondere Art und Anzahl einzunehmender Arzneimitteleinheiten und deren Einnahmezeit.

Entsprechend können im Zuge einer anderen Weiterbildung des erfindungsgemäßen Verfahrens in eine patientenspezifische Verpackung gerade solche Arzneimitteleinheiten eingepackt werden, die von einem bestimmten Patienten zu einer bestimmten Zeit eingenommen werden sollen. Die entsprechenden Informationen können auf der patientenspezifischen Verpackung angebracht, insbesondere aufgedruckt werden.

Beim Ansteuern des Dosier- und Verpackungsautomaten kann weiterhin vorgesehen sein, dass automatisch der Einnahmezeitpunkt mit der Verwendbarkeitszeit verglichen wird und dass in Abhängigkeit von dem Vergleich ein entsprechendes Steuersignal ausgelöst wird, um zu verhindern, dass Arzneimitteleinheiten abgepackt werden, die zum Einnahmezeitpunkt nicht mehr verwendbar sind.

Weiterhin kann vorgesehen sein, dass der patientenspezifische Datensatz elektronische Daten umfasst, welche in die automatische Ablaufsteuerung des Verfahrens durch ein geeignetes Datenträgermedium, wie eine Rechnernetzleitung, insbesondere des Internet, in Form von Auftragsdaten eingegeben werden. Die entsprechenden Daten können also beispielsweise extern erzeugt und zur Ablaufsteuerung des Verfahrens über das genannte Rechnernetz verfügbar gemacht werden, was insbesondere auch drahtlos geschehen kann.

Insbesondere kann vorgesehen sein, dass die genannten (Auftrags-)Daten, d.h. die patientenspezifischen Medikationsdaten von einem Abnehmer der patientenspezifischen Verpackung durch ein geeignetes Datenüberträgermedium, wie das Internet, übermittelt werden.

Wie bereits ausgeführt wurde, beinhaltet das erfindungsgemäße Verfahren optional den Schritt eines Abgleichens der patientenspezifischen Verpackung, insbesondere deren Inhaltes, mit dem patientenspezifischen Medikationsdatensatz. Dabei kann in Weiterbildung des erfindungsgemäßen Verfahrens vorgesehen sein, dass der Abgleich ein visueller Abgleich ist, insbesondere unter Nutzung einer Bild- oder Mustererkennung, wozu die Abgleich- und Prüfstation des Umpacksystems entsprechend ausgebildet ist.

Im Zuge des erfindungsgemäßen Verfahrens werden bei den einzelnen Verfahrensschritten, das heißt bei der Entnahme der Arzneimitteleinheiten aus der ersten Verpackung, der Übergabe an den Dosier- und Verpackungsautomaten, beim Ausgeben/Verpacken der Arzneimitteleinheiten und/oder beim Abgleichen der Automaten-Ausgabedaten und/oder der patientenspezifischen Verpackung mit dem patientenspezifischen Medikationsdatensatz jeweils entsprechende Datensätze und/oder Steuersignale erzeugt, insbesondere in elektronischer Form. Um eine lückenlose Dokumentation des Verfahrensablaufs beim Umpacken von Arzneimitteln zu ermöglichen, sieht eine hierauf aufsetzende weitere Ausgestaltung des erfindungsgemäßen Verfahrens vor, dass die erzeugten Datensätze und/oder Steuersignale archiviert werden, insbesondere durch Speichern in einem geeigneten elektronischen Datenspeicherungssystem.

Wie bereits erwähnt, schafft ein weiteres Beispiel der vorliegenden Erfindung ein Arzneimittelumpacksystem, insbesondere zum Durchführen des erfindungsgemäßen Verfahrens, welches eine Abfolge von entsprechend ausgebildeten Stationen umfasst. Diese sind in Weiterbildung des erfindungsgemäßen Systems mit einer übergeordneten, insbesondere rechnergesteuerten Ablaufsteuerungseinheit daten- und steuerungstechnisch verbunden. In diesem Zusammenhang ist der Begriff einer "daten- und steuerungstechnischen Verbindung" dahingehend zu verstehen, dass über eine derartige Verbindung ein Austausch von Daten bzw. Datensätzen und/oder Steuersignalen zwischen den einzelnen Stationen und insbesondere auch mit der Ablaufsteuerungseinheit ermöglicht ist. Eine derartige Verbindung kann insbesondere drahtlos oder drahtgebunen sein.

Zum Zwecke der bereits erwähnten Archivierung von Daten bzw. Datensätzen und Steuersignalen kann das erfindungsgemäße System im Zuge einer besonderen Weiterbildung eine elektronische Datenspeichereinheit aufweisen, die vorteilhafterweise in daten- und signaltechnischer Verbindung mit der Ablaufsteuerungseinheit steht. Die genannte Datenspeichervorrichtung dient zum Speichern von elektronischen Datensätzen und Steuersignalen, die an den einzelnen Stationen des erfindungsgemäßen Systems erzeugt werden, wie weiter oben ausgeführt.

Zum Übermitteln der patientenspezifischen Medikationsdaten sieht eine andere Weiterbildung des erfindungsgemäßen Systems vor, dass eine Rechnernetzanbindung, insbesondere eine Internetanbindung vorhanden ist, über welche die genannten Daten insbesondere zu der Ablaufsteuerungseinheit übertragen werden können. Die genannte Übertragung kann dabei entweder direkt zu der Ablaufsteuerungseinheit erfolgen, oder es kann wenigstens eine weitere, zwischengeschaltete Rechnereinheit vorgesehen sein, welche die genannten Daten empfängt, sammelt und nach einer gegebenenfalls erforderlichen Vorverarbeitung als Auftragsdaten an die Ablaufsteuerungseinheit weiterleitet

Der Dosier- und Verpackungsautomat ist bei einer Weiterbildung des erfindungsgemäßen Systems vorteilhafterweise derart ausgebildet, dass er für jedes Arzneimittel bzw. die entsprechenden Arzneimitteleinheiten einen eigenen Vorratsbehälter aufweist. Wenn also ein bestimmtes Arzneimittel bzw. eine oder mehrere entsprechende Arzneimitteleinheiten in eine patientenspezifische Verpackung einzupacken sind, wird der zugehörige Vorratsbehälter des Dosierund Verpackungsautomaten angesteuert, um die erforderliche Menge an Arzneimitteleinheiten auszugeben, vorzugsweise in Kunststoffbeutel oder Blister.

In diesem Zusammenhang ist es von besonderem Vorteil, wenn - wie weiter oben ausgeführt - an sich identische und somit gleichartige Arzneimitteleinheiten, die jedoch arzneimittelrechtlich zu unterschiedlichen Arzneimitteln gehören, mit einer übergeordneten, gemeinsamen Kennzeichnung versehen sind, so dass bei der Ansteuerung des Dosier- und Verpackungsautomaten alternativ auf mehrere Vorratsbehälter zugegriffen werden kann, die jeweils der übergeordneten, gemeinsamen Kennzeichnung zugeordnete und somit gleichartige Arzneimitteleinheiten enthalten. So können beispielsweise zwei Vorratsbehälter des Dosier- und Verpackungsautomaten gleichartige Arzneimitteleinheiten enthalten, von denen jedoch die einen aus einer N1-Verpackung und die anderen aus N2-Verpackung entnommen wurden, so dass sich die jeweils zugehörigen arzneimittelspezifischen Datensätze in der Pharmazentralnummer (PZN) unterscheiden. Wenn nun einem bestimmten Patienten eigentlich Arzneimitteleinheiten aus der genannten N1-Verpackung verordnet wurden, diese jedoch aus einem bestimmten Grund nicht verfügbar sind (beispielsweise weil die entsprechende Verwendbarkeitszeit zu kurz oder der betreffende Behälter gerade leer ist), können statt dessen aufgrund der übergeordneten gemeinsamen Kennung, die vorzugsweise in den betreffenden arzneimittelspezifischen Datensätzen enthalten oder in geeigneter Weise damit verknüpft ist, alternativ Arzneimitteleinheiten aus der genannten N2-Verpackung ausgegeben werden, wenn diese im System verfügbar sind, ohne dass sich hierdurch im Hinblick auf die zu erzeugende patientenspezifische Verpackung bzw. für den Patienten selbst irgendwelche wahrnehmbaren Änderungen ergeben würden. Dadurch erhöht sich, wie bereits ausgeführt wurde, die Verwendungsflexibilität des erfindungsgemäßen Arzneimittelumpacksystems.

Die Steuerung der vorstehend beschriebenen Verfahrensabläufe, insbesondere auf einem ebenfalls weiter oben beschriebenen Arzneimittelumpacksystem, erfolgt vorzugsweise durch Rechnersteuerung, wozu mindestens eine zentrale oder mehrere dezentrale Rechnersysteme als Steuerungseinheiten vorgesehen sein können. Diese arbeiten nach Maßgabe von Programmanweisungen, welche auf einem geeigneten maschinenlesbaren Datenträger gespeichert sind, wie einer CD-ROM, einer DVD, einem Flash-Speicher oder einem anderen geeigneten Datenträgermedium. Die genannten Programmanweisungen werden zur Ausführung auf den genannten Rechner- oder Steuerungseinheiten verfügbar gemacht und dienen insbesondere dazu, die während des erfindungsgemäßen Verfahrens erzeugten elektronischen Datensätze und/oder Steuersignale zu erzeugen, zu archivieren und zu visualisieren.

Eine besonders vorteilhafte Weiterbildung der Erfindung ergibt sich, wenn ein erfindungsgemäßes Computerprogrammprodukt derart weitergebildet ist, dass es auf einem Datenträger gespeicherte zweite Programmanweisungen aufweist, bei deren Ausführung eine Rechnernetzschnittstelle, insbesondere eine Internetschnittstelle, aktiviert wird, um zumindest einen patientenspezifischen Medikationsdatensatz über ein Rechnernetz bzw. das Internet zu dem erfindungsgemäßen Arzneimittelumpacksystem zu übertragen, wobei die Übertragung direkt oder indirekt, das heißt optional über eine Anzahl weiterer zwischengeschalteter Rechnersysteme erfolgen kann. Auf diese Weise ist ein Abnehmer der erfindungsgemäß erzeugten patientenspezifischen Verpackungen in der Lage, diese über das Internet zu spezifizieren und zu bestellen, so dass sich eine besonders rationelle Ausgestaltung des erfindungsgemäßen Verfahrens ergibt.

Insbesondere durch die umfassende Dokumentationsfähigkeit des Verfahrensablaufs beim Umpacken von Arzneimitteln erlaubt es die vorliegende Erfindung einem Pharmaunternehmer, die hohen Anforderungen des im Arzneimittelbereich geltenden "good manufacturing practice" (GMP) zu erfüllen.

Weitere Eigenschaften und Vorteile der vorliegenden Erfindung ergeben sich aus der nachfolgenden Beschreibung von Ausführungsbeispielen anhand der Zeichnung. Es zeigt:
Figur 1 ein schematisches Blockschaltbild eines erfindungsgemäßen Arzneimittelumpacksystems; und
Figur 2 ein Flussdiagramm einer Ausgestaltung des erfindungsgemäßen Verfahrens zum Umpacken von Arzneimitteln.

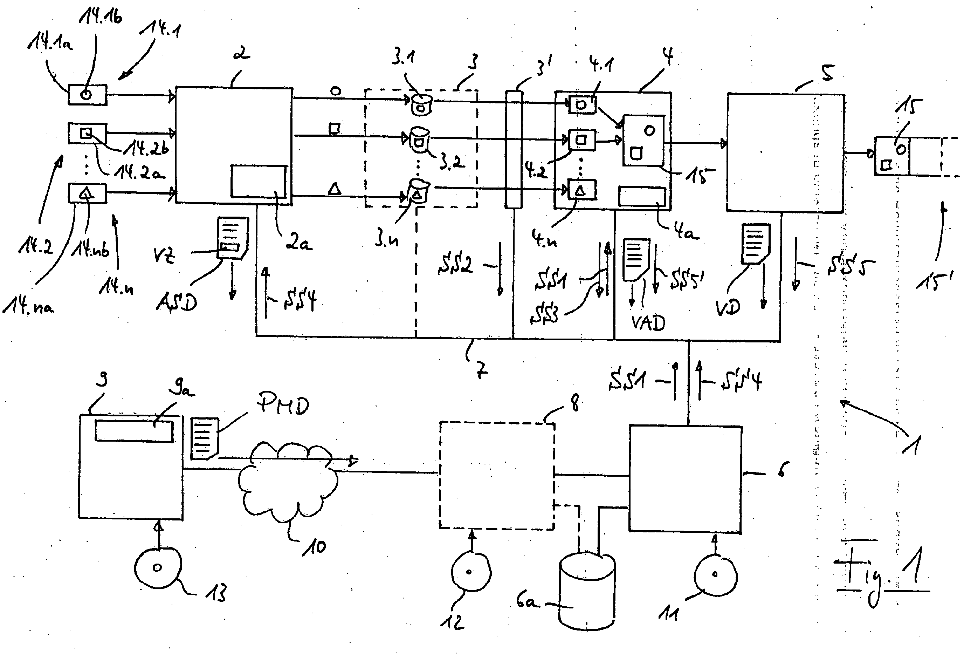

Figur 1 zeigt ein schematisches Blockschaltbild einer exemplarischen Ausgestaltung des erfindungsgemäßen Arzneimittelumpacksystems 1. Das System umfasst eine Entnahme- und Erfassungsstation 2, eine optionale Bevorratungsstation 3 (in Figur 1 gestrichelt dargestellt), eine Abfüll- und Verpackungsstation 4 sowie eine Abgleich- und Prüfstation 5. Die vorstehend genannten Stationen 2 - 5 sind in Richtung des Prozessablaufs beim Umpacken der Arzneimittel hintereinander angeordnet bzw. "in Reihe beschaltet". Zwischen der Entnahme- und Erfassungsstation 2 bzw. der optionalen Bevorratungsstation 3 einerseits und der Abfüll- und Verpackungsstation 4 andererseits ist zudem noch eine Übergabeschnittstelle 3' angeordnet. Die Entnahmeund Erfassungsstation 2, die Übergabeschnittstelle 3', die Abfüll- und Verpackungsstation 4, die Abgleich- und Prüfstation 5 sowie optional die Bevorratungsstation 3. sind signal- und steuerungstechnisch mit einer Ablaufsteuerungseinheit 6 verbunden, beispielsweise über das in Figur 1 exemplarisch dargestellte Bussystem 7. Wie der Fachmann erkennt, können in diesem Zusammenhang auch andere Netztopologien und/oder drahtlose Verbindungen zum Einsatz kommen.

Die Ablaufsteuerungseinheit 6 kann als Rechnersystem herkömmlicher Art ausgebildet sein und entsprechend Ein- und Ausgabeeinrichtungen, Prozessormittel, Speichermittel und dergleichen aufweisen, was in Figur 1 nicht explizit dargestellt ist. Mit der Ablaufsteuerungseinheit 6 ist eine Speichereinheit 6a verbunden, auf deren Funktion weiter unten noch genauer eingegangen wird. Die Ablaufsteuerungseinheit 6 ist über ein in Figur 1 gestrichelt dargestelltes, optionales Rechnersystem 8 mit einem weiteren Rechnersystem 9 verbunden, bei dem es sich insbesondere um einen herkömmlichen PC mit entsprechenden Peripheriegeräten handeln kann, was in Figur 1 ebenfalls nicht explizit dargestellt ist. Die Verbindung zwischen der Ablaufsteuerungseinheit 6 bzw. dem optionalen Rechnersystem 8 einerseits und dem weiteren Rechnersystem 9 andererseits erfolgt zumindest streckenweise über ein öffentliches Rechnernetz 10, wie das Internet. Die Rechnersysteme 6, 8, 9 sind mittels geeigneter Computerprogrammprodukte, das heißt durch das Ausführen von Programmanweisungen, die auf entsprechenden Datenträgermedien 11, 12 bzw. 13 gespeichert sind, für die Ihnen jeweils zugedachten Funktionen eingerichtet, worauf weiter unten noch genauer eingegangen wird.

Im Falle seines Vorhandenseins ist auch das optionale Rechnersystem 8 mit der Speichereinheit 6a verbunden, was in Figur 1 mittels einer entsprechenden gestrichelten Verbindungslinie dargestellt ist.

Nachfolgend werden die einzelnen Funktionen des erfindungsgemäßen Arzneimittelumpacksystems 1 bzw. dessen vorstehend aufgeführter Bestandteile unter Bezugnahme auf einen exemplarischen Verfahrensablauf beim Umpacken von Arzneimitteln näher beschrieben:
Die Arzneimittel werden an der Entnahme- und Erfassungsstation 2 bereitgestellt. In Figur 1 sind in diesem Zusammenhang exemplarisch eine Anzahl unterschiedlicher Arzneimittel 14.1, 14.2, ..., 14.n dargestellt. Diese umfassen jeweils eine erste Verpackung 14.1a, 14.2a, ..., 14.na, in der jeweils eine bestimmte Anzahl von Arzneimitteleinheiten 14.1b, 14.2b, ..., 14.nb enthalten sind, beispielsweise in Form von Tabletten, Pastillen, Kapseln oder dergleichen. In der Darstellung gemäß Figur 1 sind die Arzneimitteleinheiten 14.1b, 14.2b, ..., 14.nb unterschiedlicher Arzneimittel 14.1, 14.2, ..., 14.n jeweils durch unterschiedliche geometrische Symbole (Kreis, Quadrat, Dreieck) dargestellt, um die "Lesbarkeit" der Figur zu der Darstellung zu erhöhen. Die ersten Verpackungen 14.1a, 14.2a, ..., 14.na können jeweils noch in einer geeigneten Umverpackung enthalten sein, was in Figur 1 nicht explizit gezeigt ist. Während die ersten Verpackungen 14.1a, 14.2a, ..., 14.na vorzugsweise als Blisterverpackungen ausgebildet sind, handelt es sich bei den Umverpackungen insbesondere um Faltschachteln aus Kartonmaterial oder dergleichen, ohne das die Erfindung hierauf beschränkt wäre.

Wie in der Figur 1 durch entsprechende Pfeile symbolisiert ist, werden die Arzneimittel 14.1, 14.2, ..., 14.n zu der Entnahme- und Erfassungsstation 2 gefördert, was automatisch über geeignete Fördereinrichtungen (in Figur 1 nicht gezeigt) und/oder manuell geschehen kann.

An der Entnahme- und Erfassungsstation 2 werden die einzelnen Arzneimitteleinheiten 14.1b, 14.2b, ... , 14.nb aus den jeweiligen Verpackungen 14.1a, 14.2a, ..., 14.na entnommen. Im Falle einer Blisterverpackung kann dies ein Entblistern einzelner Arzneimitteleinheiten, das heißt ein Herausdrücken der Arzneimitteleinheiten aus der entsprechenden Blisterverpackung beinhalten. Bestimmte Arzneimittel, die aufgrund von arzneimittelrechtlichen Bestimmungen nicht entpackt (z. B. entblistert) werden dürfen, wie Antibiotika oder dergleichen, werden entsprechend zu Arzneimitteleinheiten vereinzelt, indem beispielsweise eine entsprechende Blisterverpackung zwischen den Arzneimitteleinheiten getrennt oder geschnitten wird, um individuell verpackte Arzneimitteleinheiten zu erzeugen. Hinter der Entnahme- und Erfassungsstation 2 (rechts in Figur 1) liegen dann individuelle Arzneimitteleinheiten 14.1b, 14.2b, ..., 14.nb vor, was in Figur 1 wiederum durch die entsprechenden, "nackten" Symbole dargestellt ist.

Auch das erwähnte Zerschneiden von Verpackungen zum Erzeugen individuell verpackter Arzneimitteleinheiten kann im Rahmen der vorliegenden Erfindung maschinell und/oder manuell erfolgen. Dies trifft zusätzlich auch für ein eventuelles Öffnen der (Karton-)Umverpackungen für die einzelnen Arzneimittel 14.1, 14.2, ..., 14.n zu.

Weiterhin dient die Entnahme- und Erfassungsstation 2 auch zum Erfassen eines arzneimittelspezifischen Datensatzes ASD für das entnommene Arzneimittel 14.1, 14.2, ..., 14.n. In Figur 1 ist ein solcher arzneimittelspezifischer Datensatz ASD symbolisch dargestellt; jedoch wird im Rahmen der vorliegenden Erfindung für jedes Arzneimittel 14.1, 14.2, ..., 14.n ein solcher Datensatz erzeugt. Bei dem arzneimittelspezifischen Datensatz ASD handelt es sich um einen elektronischen Datensatz, der durch ein Rechnersystem, wie die Ablaufsteuerungseinheit 6, lesbar ist. Der arzneimittelspezifische Datensatz ASD wird für jedes Arzneimittel vorteilhafterweise unmittelbar beim Entnehmen aus der ersten Verpackung 14.1a, 14.2a, ..., 14.na erzeugt. Allerdings ist die vorliegende Erfindung nicht darauf beschränkt, dass das Entnehmen der Arzneimittel und die Erfassung zum Erzeugen des arzneimittelspezifischen Datensatzes ASD an einem Ort, das heißt an einer gemeinsamen Station 2 erfolgt, wie dies in Figur 1 lediglich exemplarisch dargestellt ist.

Der arzneimittelspezifische Datensatz ASD gelangt über das Bussystem 7 zu der Ablaufsteuerungseinheit 6 und wird dort in einem (nicht gezeigten) Arbeitsspeicher, vorzugsweise jedoch in der Speichereinheit 6a gespeichert.

Der arzneimittelspezifische Datensatz ASD enthält vorzugsweise zumindest eines oder mehrere der nachfolgend aufgeführten Daten, wobei es sich bei der nachfolgenden Aufzählung um eine nicht abschließende Aufzählung handelt, welche der Fachmann bei Bedarf leicht ergänzen oder erweitern wird:
- Art des Arzneimittels, insbesondere angegeben in Form der entsprechenden Pharmazentralnummer (PZN);
- Anzahl der aus der Verpackung entnommenen und/oder der fehlerhaften Arzneimitteleinheiten;
- Haltbarkeitsangaben, insbesondere das Verwendbarkeitsdatum des betreffenden Arzneimittels;
- Aktualisierungsstand (Datum) der Produktinformationen gemäß Packungsbeilage.

Insbesondere wird in diesem Zusammenhang bei der Erzeugung des arzneimittelspezifischen Datensatzes ASD ein auf der Verpackung 14.1a, 14.2a, ..., angegebener Identifizierungscode (Barcode) gelesen bzw. gescannt. Weitere Daten können an der Entnahme- und Erfassungsstation 2 zum Erzeugen des arzneimittelspezifischen Datensatzes ASD über weitere dort vorhandene Eingabemittel 2a erfasst werden. In der Darstellung gemäß Figur 1 umfassen die genannten Eingabemittel 2a demnach insbesondere ein Barcode-Lesegerät (Scanner) sowie eine Tastatur zum manuellen Erfassen des Aktualisierungsstands der Produktinformationen oder dergleichen.

Darüber hinaus wird, wie in Figur 1 dargestellt, jedem arzneimittelspezifischen Datensatz ASD automatisch eine Verwendbarkeitszeit in Abhängigkeit von der Entnahmezeit zugewiesen, was entweder durch geeignete Zuweisungsmittel (nicht gezeigt) an der Station selbst oder durch die Ablaufsteuereinheit 6 nach Erhalt des Datensatzes ASD geschehen kann. Bei der gezeigten Ausgestaltung der Erfindung ist die Verwendbarkeitszeit, das heißt eine entsprechende Zeitangabe Teil des arzneimittelspezifischen Datensatzes ASD bei dessen Erzeugung und innerhalb diesem durch das Bezugszeichen VZ bezeichnet. Die Verwendbarkeitszeit VZ gibt an, innerhalb welches Zeitraums ein entnommenes Arzneimittel 14.1, 14.2, ..., 14.n, das heißt die betreffenden Arzneimitteleinheiten 14.1b, 14.2b, ..., 14.nb einem Patienten verabreicht werden kann bzw. können, wobei der entsprechende Zeitraum im Zeitpunkt der Entnahme der Arzneimitteleinheiten aus der Verpackung 14.1a, 14.2a, ..., 14.na beginnt. Die Verwendbarkeitszeit VZ orientiert sich dabei an einer für die entnommenen Arzneimittel (einheitlich) definierten Stabilitätszeit, welche angibt, innerhalb welcher Zeiträume ein bestimmtes Arzneimittel nach dem Entpacken im Wesentlichen unverändert vorliegt (beispielsweise in Bezug auf Feuchtigkeitsaufnahme aus der Umgebung oder dergleichen).

Anhand des arzneimittelspezifischen Datensatzes ASD lässt sich also lückenlos dokumentieren, wie viele Einheiten eines bestimmten Arzneimittels an der Entnahme- und Erfassungsstation 2 entnommen wurden und bis wann die betreffenden Arzneimitteleinheiten 14.1b, 14.2b, ..., 14.nb verwendet werden dürfen. Zusätzlich kann der arzneimittelspezifische Datensatz ASD noch die weiter oben detailliert erläuterten Informationen und gegebenenfalls zusätzliche Einträge enthalten. Selbstverständlich sollte die Verwendbarkeitszeit VZ nicht länger als die Haltbarkeitsdauer des betreffenden Arzneimittels sein.

Mittels des arzneimittelspezifischen Datensatzes ASD stehen die genannten Informationen in dem Arzneimittelumpacksystem 1 insbesondere der Ablaufsteuerungseinheit 6 zur Verfügung und können zu Zwecken der Ablaufsteuerung verwendet werden, worauf weiter unten noch genauer eingegangen wird.

Die entnommenen Arzneimitteleinheiten 14.1b, 14.2b, ..., 14.nb gelangen von der Entnahme- und Erfassungsstation 2 zu der optionalen Bevorratungsstation 3, welche eine Anzahl von Vorratsbehältern 3.1, 3.2, ..., 3.n aufweist, die zum Lagern der entnommenen Arzneimitteleinheiten 14.1b, 14.2b, ..., 14.nb ausgebildet sind. Dabei erfolgt die Lagerung der Arzneimitteleinheiten 14.1b, 14.2b, ..., 14.nb in der Bevorratungsstation 3 nach Arzneimitteln getrennt in entsprechenden Vorratsbehältern 3.1, 3.2, ..., 3.n, welche ihrer Art nach insbesondere zur Sicherung der Arzneimittelstabilität ausgebildet sind, beispielsweise als getönte Glasbehälter mit Schraubdeckel, in den gegebenenfalls ein Trocknungsmittel eingebracht ist.

Wie bereits mehrfach erwähnt wurde, ist das Vorhandensein der Bevorratungsstation 3 innerhalb des Arzneimittelumpacksystems 1 nicht zwingend erforderlich; vielmehr können die entnommenen Arzneimitteleinheiten 14.1b, 14.2b, ..., 14.nb auch direkt von der Entnahme- und Erfassungsstation 2 über die Übergabeschnittstelle 3' an die Abfüll- und Verpackungsstation 4 übergeben werden, insbesondere wenn bei Letzterer ein Engpass an einem bestimmten Arzneimittel besteht. Die Abfüll- und Verpackungsstation 4 ist gemäß der Ausgestaltung in Figur 1 in Form eines Dosier- und Verpackungsautomaten ausgebildet, der seine eigene Steuereinheit 4a aufweist, welche über das Bussystem 7 von der Ablaufsteuerungseinheit 6 des Arzneimittelumpacksystem 1 angesprochen oder angesteuert wird.

Der Dosier- und Verpackungsautomat 4 ist als solcher bekannt und weist eine größere Anzahl von Vorrats- oder Zuführbehältern 4.1, 4.2, ..., 4.n auf, wobei für jedes zu dosierende bzw. zu verpackende Arzneimittel 14.1, 14.2, ..., 14.n ein eigener Behälter 4.1, 4.2, ..., 4.n vorgesehen ist, was in Figur 1 wiederum durch entsprechende geometrische Symbole verdeutlicht wird. Nach Maßgabe der Steuereinheit 4a ist der Dosier- und Verpackungsautomat 4 in der Lage, Arzneimitteleinheiten 14.1b, 14.2b, ..., 14.nb, welche in den entsprechenden Behältern 4.1, 4.2, ..., 4.n enthalten sind, auszugeben und in einer spezifischen Verpackung 15, insbesondere einem Blisterbeutel oder Folienbeutel, einzupacken. Dies ist in Figur 1 schematisch dargestellt, wobei vorliegend exemplarisch eine Arzneimitteleinheit 14.1 b aus dem Behälter 4.1 sowie eine Arzneimitteleinheit 14:2b aus dem Behälter 4.2 in der patientenspezifischen Verpackung 15 eingepackt werden. Dies geschieht - wie gesagt - nach Maßgabe der Steuereinheit 4a, welche die betreffenden Behälter 4.1, 4.2 auswählt und entsprechend für das Ausgeben der Arzneimitteleinheiten 14.1b, 14.2b in die patientenspezifische Verpackung 1.5 sorgt.

Die Steuereinheit 4a wird in diesem Zusammenhang durch die übergeordnete Ablaufsteuerungseinheit 6 des Arzneimittelumpacksystems 1 angesteuert, welche die erforderlichen Ansteuerungsinformationen einem weiteren elektronischen Datensatz entnimmt, der vorliegend als Patientenmedikationsdatensatz oder als Patientenmedikationsdaten PMD bezeichnet wird. Der Patientenmedikationsdatensatz PMD enthält Angaben, die es ermöglichen, eine Verknüpfung zwischen Patienten bzw. Patientendaten und von diesem Patienten einzunehmenden Arzneimitteln, das heißt entsprechenden Arzneimitteldaten herzustellen. Beispielsweise kann vorgesehen sein, dass der patientenspezifische Medikationsdatensatz PMD wenigstens die folgenden Daten enthält:
- Identifikationsdaten des Patienten, insbesondere dessen Namen, Alter, Geschlecht, Wohnort, Behandlungsort, Krankheitsbild oder dergleichen;
- Medikationsdaten, insbesondere betreffend die Art und Anzahl einzunehmender Arzneimitteleinheiten sowie deren Einnahmezeitpunkte, z. B. morgens 2 Tabletten A, mittags eine Kapsel B usw.

Der patientenspezifische Medikationsdatensatz PMD bzw. eine Mehrzahl entsprechender Datensätze steht der Ablaufsteuerungseinheit 6 im Rahmen der vorliegenden Erfindung als Auftragsdaten zur Verfügung, so dass diese entsprechend den Dosier- und Verpackungsautomaten 4 bzw. dessen Steuereinheit 4a ansteuern kann, um Arzneimitteleinheiten 14.1b, 14.2b in eine patientenspezifische Verpackung 15 einzupacken, wie oben bereits beschrieben.

Beim Ausgeben bzw. Verpacken der Arzneimitteleinheiten 14.1b, 14.2b generiert der Dosier- und Verpackungsautomat 4 entsprechende verpackungsspezifische Ausgabedaten VAD in elektronischer Form und liefert diese über das Bussystem 7 zu der Ablaufsteuerung 6. Die verpackungsspezifischen Ausgabedaten VAD geben an, welche und wie viele Arzneimitteleinheiten der Automat 4 in Reaktion auf einen entsprechenden Auftrag bzw. die entsprechende Ansteuerung durch die Ablaufsteuerungseinheit 6 ausgeben und verpackt hat oder zu haben "glaubt". Die Ausgabedaten VAD können insbesondere dann von den Auftragsdaten (Patientenmedikationsdaten PMD) abweichen, wenn diese inzwischen geändert (z. B. storniert) wurden, nachdem der Dosier- und Verpackungsautomat 4 bereits angesteuert worden war.

Nachfolgend wird das Erzeugen der patientenspezifischen Medikationsdaten PMD und deren Übertragung zu der Ablaufsteuerungseinheit 6 des Arzneimittelumpacksystems 1 genauer beschrieben:
Gemäß der beispielhaften Ausgestaltung in Figur 1 werden die patientenspezifischen Medikationsdaten PMD an dem zusätzlichen Rechnersystem 9 erzeugt, bei dem es sich um ein Standard-PC-System handeln kann. Das Rechnersystem 9 ist örtlich nicht an den Standort des restlichen Arzneimittelumpacksystems 1 gebunden, da die Verbindung - wie bereits angesprochen - insbesondere über das Internet 10 erfolgen kann.

Auf dem Rechnersystem 9 werden mittels eines geeigneten Datenträgermediums 13 bzw. mittels eines entsprechenden Computerprogrammprodukts, welches auf dem Datenträgermedium 13 enthalten oder gespeichert ist, Programmanweisungen implementiert und ausgeführt, welche es einem Benutzer des Rechnersystems 9 ermöglichen, dem patientenspezifischen Medikationsdatensatz PMD in einfacher Weise zu erzeugen und an das restliche Arzneimittelumpacksystem 1, insbesondere dessen Ablaufsteuerungseinheit 6, zu übermitteln, insbesondere via Internet 10. Die genannten Programmanweisungen, welche auf dem Rechnersystem 9 implementiert bzw. ausgeführt werden, schaffen dort vorzugsweise eine grafische Benutzeroberfläche 9a (graphical user interface - GUI), mittels der ein Benutzer patientenspezifische Medikationsdatensätze PMD über Standardeingabemittel des Rechnersystems 9, wie eine Tastatur, Maus oder dergleichen, in einfacher Weise erzeugen und übertragen kann. Insbesondere können es die auf dem Rechnersystem 9 ablaufenden Programmanweisungen ermöglichen, patientenspezifische Medikationsdatensätze PMD durch einfaches Auswählen und Anklicken von Daten in entsprechenden Patienten-, Arzneimittel-, Mengen- und Zeitmenüs auszuwählen. Beispiele für vergleichbare Anwendungssoftware sind dem Fachmann bekannt, so dass vorliegend darauf nicht weiter einzugehen ist.

Nach Erzeugen des exemplarischen Patientenmedikationsdatensatzes PMD wird dieser via Internet 10 zu dem restlichen Arzneimittelumpacksystem 1 übertragen, was in Figur 1 durch einen entsprechenden Pfeil symbolisch dargestellt ist.

Gemäß der Ausgestaltung in Figur 1 umfasst das (restliche) Arzneimittelumpacksystem 1 ein optionales weiteres Rechnersystem 8, welches den Patientenmedikationsdatensatz PMD über das Internet 10 empfängt und welches vorliegend zur Erfassung eingehender Aufträge zur Arzneimittelumpackung dient. Das optionale Rechnersystem 8 kann die eingehenden Aufträge bzw. die zugehörigen Patientenmedikationsdatensätze PMD verwalten, speichern, aufbereiten oder dergleichen und führt zu diesem Zweck entsprechende Programmanweisungen durch, welche auf dem Datenträgermedium 12 gespeichert sind. Anschließend überträgt das optionale Rechnersystem 8 die gegebenenfalls aufbereiteten Patientenmedikationsdatensätze PMD zu der Ablaufsteuerungseinheit 6 - entweder direkt oder mittels Zwischenspeicherung in der Speichereinheit 6a, auf welche das optionale Rechnersystem 8 und die Ablaufsteuerungseinheit 6 gemäß Figur 1 gemeinsam zugreifen können.

Der Ablaufsteuerungseinheit 6 stehen auf dieser Weise sowohl patientenspezifische Medikamentationsdatensätze PMD als auch arzneimittelspezifische Datensätze ASD zur Verfügung. Die Patientenmedikamentationsdatensätze PMD enthalten Angaben darüber, welcher Patient zu welchem Zeitpunkt welche Arzneimittel (das heißt eine Anzahl von Arzneimitteleinheiten eines bestimmten Arzneimittels) erhalten soll. Die arzneimittelspezifischen Datensätze ASD geben an, wie viele Einheiten eines bestimmten Arzneimittels innerhalb des Systems 1 verfügbar sind und bis wann diese Arzneimitteleinheiten jeweils umgepackt werden dürfen (Verwendungszeit VZ).

Die Ablaufsteuerungseinheit 6 kann nun den Dosier- und Verpackungsautomaten 4 bzw. dessen Steuereinheit 4a mittels geeigneter Steuersignale SS1 anweisen, Arzneimitteleinheiten 14.1b, 14.2b aus den entsprechenden Behältern 4.1, 4.2 gemäß gegebenen Patientenmedikamentationsdaten PMD in die patientenspezifische Verpackung 15 einzupacken, wie weiter oben bereits beschrieben wurde. In diesem Zusammenhang dient die Übergabeschnittstelle 3' dazu sicherzustellen, dass in dem System 1, das heißt in der Ablaufsteuerungseinheit 6 jederzeit bekannt ist, welches Arzneimittel bzw. welche Art von Arzneimitteleinheiten sich in einem gegebenen Behälter 4.1, 4.2, ..., 4.n des Dosier- und Verpackungsautomatens 4 befindet. Mit anderen Worten: Durch die Übernahmeschnittstelle 3' wird eine datentechnische Verknüpfung zwischen den Arzneimitteleinheiten 14.1b, 14.2b, ..., 14.nb und den Behältern 4.1, 4.2, ..., 4.n des Dosier- und Verpackungsautomatens 4 hergestellt und in Form geeigneter Steuersignale SS2 zu der Ablaufsteuerungseinheit 6 übertragen. Bei eine speziellen Ausgestaltung des gezeigten Arzneimittelumpacksystems 1 kann dies beispielsweise dadurch erfolgen, dass ein den entnommenen Arzneimitteleinheiten 14.1b, 14.2b, ..., 14.nb jeweils zugeordneter Identifizierungscode (z. B. ein Barcode oder dergleichen, der auf dem entsprechenden Vorratsbehälter 3.1, 3.2, ..., 3.n angebraucht sein kann) an der Übergabeschnittstelle 3' gelesen und mit einem jeweiligen Identifizierungscode der Behälter 4.1, 4.2, ..., 4.n logisch verknüpft wird, wenn Einheiten eines bestimmten Arzneimittels in einen bestimmten Behälter eingefüllt werden. Diese Informationen werden der Ablaufsteuerungseinheit 6 durch das Steuersignal SS2 mitgeteilt, so dass diese entsprechend "weiß", welche Arzneimittel an welchem Ort und gegebenenfalls in welcher Menge innerhalb des Dosier- und Verpackungsautomaten 4 vorhanden sind.

Beim Dosieren und Verpacken innerhalb des Dosier- und Verpackungsautomaten 4 selbst stellt dessen Steuereinheit 4a insbesondere sicher, dass der Automat keine leeren Behälter 4.1, 4.2, ..., 4.n aufweist, wenn aus diesen Arzneimitteleinheiten entnommen werden sollen. Sollte dies dennoch der Fall sein, was im Betrieb des erfindungsgemäßen Umpacksystems 1 durchaus regelmäßig geschieht, oder im Falle sonstiger Störungen des Dosier- und Verpackungsautomaten 4 gibt die Steuereinheit 4a entsprechende Steuersignale SS3 an die Ablaufsteuerungseinheit 6 aus, so dass entsprechende Gegenmaßnahmen eingeleitet werden können, was dem Fachmann an sich bekannt ist, so dass aufgetretene Störungen beseitigt werden.

Durch Ihre Kenntnis sowohl der arzneimittelspezifischen Datensätze ASD als auch der Patientenmedikationsdatensätze PMD ist die Ablaufsteuerungseinheit 6 erfindungsgemäß insbesondere in der Lage sicherzustellen, dass gemäß einem Patientenmedikationsdatensatz PMD keine Arzneimitteleinheiten in einer patientenspezifischen Verpackung 15 eingepackt werden, deren vorgeschriebener Einnahmezeitpunkt hinter der Verwendbarkeitszeit VZ liegt, welche in dem arzneimittelspezifischen Datensatz ASD enthalten ist. Sollte der Fall auftreten, dass ein Patientenmedikationsdatensatz PMD nach einem Arzneimittel "verlangt", dessen Arzneimitteleinheiten in dem Dosier- und Verpackungsautomaten 4 nur mit einer zu kurzen Verwendbarkeitzeit VZ gemäß dem zugehörigen arzneimittelspezifischen Datensatz ASD vorhanden sind, erzeugt die Ablaufsteuerungseinheit 6 ein entsprechendes Steuersignal SS4, welches in dem gezeigten Arzneimittelumpacksystem 1 in vielfältiger Weise verwendbar ist, beispielsweise zum Anzeigen eines Fehlerzustands an eine System-Bedienperson oder - wie in Figur 1 gezeigt - als Steuersignal für die Entnahme- und Erfassungsstation 2, um dort ein Entpacken zusätzlicher Einheiten des betreffenden Arzneimittels mit einer geeigneten neuen Verwendbarkeitszeit VZ zu veranlassen.

Eine ebenfalls optionale signal- und datentechnische Verbindung (in Figur 1 gestrichelt gezeichnet) der optionalen Bevorratungsstation 3 mit der Ablaufsteuerungseinheit 6 über das Bussystem 7 dient in diesem Zusammenhang insbesondere dazu, die Ablaufsteuerungseinheit 6 mit Informationen dahingehend zu versorgen, welche Arzneimitteleinheiten zu einem bestimmten Zeitpunkt noch an der Bevorratungsstation 3 gelagert sind. Alternativ zu der oben beschriebenen Übermittlung des Steuersignals SS4 an die Entnahme- und Erfassungsstation 2 besteht dann bei gegebener Bevorratung von Arzneimitteleinheiten mit ausreichender Verwendbarkeitszeit VZ an der Bevorratungsstation 3 die Möglichkeit, das Steuersignal SS4 abweichend von der Darstellung in Figur 1 zu der Bevorratungsstation 3 zu senden, um eine entsprechende Übergabe der betreffenden Arzneimitteleinheiten von der Bevorratungsstation 3 an den Dosier- und Verpackungsautomat 4 durch die Übergabeschnittstelle 3' zu veranlassen.

Der Dosier- und Verpackungsautomat 4 ist dazu ausgebildet, einen automatischen Abgleich der verpackungsspezifischen Ausgabedaten VAD mit dem aktuell gültigen patientenspezifischen Medikationsdatensatz PMD, d. h. den Auftragsdaten vorzunehmen. Mit anderen Worten: Der Dosier- und Verpackungsautomat 4 dient zugleich der Überprüfung, ob in einer gegebenen patientenspezifischen Verpackung 15 entsprechend den Ausgabedaten VAD diejenigen Arzneimitteleinheiten 14.1 b, 14.2b enthalten sind, welche nach dem aktuell gültigen Medikationsdatensatz PMD darin enthalten sein sollten, wobei die Überprüfung entweder nach Maßgabe der Ablaufsteuerungseinheit 6 oder direkt durch diese selbst erfolgt, welcher insbesondere die entsprechenden Patientenmedikationsdaten PMD bekannt sind. Im Falle einer Nichtkonsistenz der Daten VAD und PMD erzeugt gemäß Figur 1 der Automat 4 (oder alternativ die Steuereinheit 6) ein entsprechendes Steuersignal SS5', welches an die Ablaufsteuerungseinheit 6 übermittelt wird. Diese kann dann gegebenenfalls einen Fehlerzustand anzeigen und/oder geeignete Abhilfemaßnahmen veranlassen.

Nachfolgend sei angenommen, dass durch den Dosier- und Verpackungsautomat 4 nach Maßgabe der Ablaufsteuerungseinheit 6 - wie beschrieben - eine patientenspezifische Verpackung 15 mit einer Anzahl von Arzneimitteleinheiten 14.1b, 14.2b scheinbar korrekt erzeugt wurde, was mit der Erzeugung entsprechender verpackungsspezifischer Ausgabedaten VAD einhergeht, wie beschrieben. Der Begriff "patientenspezifische Verpackung" bezeichnet vorliegend eine Verpackungseinheit, beispielsweise in Form eines Blister- oder Folienbeutels, in der auftragsgemäß eine Anzahl von Arzneimitteleinheiten gemäß einem Patientenmedikationsdatensatz PMD eingepackt sind und die darüber hinaus im Hinblick auf den betreffenden Patient, die enthaltenen Arzneimitteleinheiten und/oder den Einnahmezeitpunkt individuell gekennzeichnet sein kann. Die hergestellte patientenspezifische Verpackung 15 wird gemäß Figur 1 nach rechts zu der Abgleich- und Prüfstation 5 gefördert, was wiederum maschinell-automatisch oder manuell geschehen kann. Die Abgleich- und Prüfstation 5 ist dazu ausgebildet, einen automatischen Abgleich der patientenspezifischen Verpackung 15, insbesondere deren Inhalts, mit dem bei der Erzeugung zugrunde gelegten patientenspezifischen Medikationsdatensatz PMD vorzunehmen. Mit anderen Worten: Die Abgleich- und Prüfstation 5 dient der Überprüfung, ob in einer gegebenen patientenspezifischen Verpackung 15 diejenigen Arzneimitteleinheiten 14.1b, 14.2b enthalten sind, welche nach dem entsprechenden Medikationsdatensatz PMD darin enthalten sein sollten, wobei die Überprüfung nach Maßgabe der Ablaufsteuerungseinheit 6 oder direkt durch diese selbst erfolgt, welcher insbesondere die entsprechenden Patientenmedikationsdaten PMD bekannt sind. Im Falle einer Nichtkonsistenz erzeugt gemäß Figur 1 die Abgleich- und Prüfstation 5 (oder alternativ die Steuereinheit 6) ein entsprechendes Steuersignal SS5, welches an die Ablaufsteuerungseinheit 6 übermittelt wird. Diese kann dann gegebenenfalls einen Fehlerzustand anzeigen und/oder geeignete Abhilfemaßnahmen veranlassen.

Die Überprüfung an der Abgleich- und Prüfstation 5 erfolgt vorteilhafter Weise zusätzlich zu dem Datenabgleich durch den Automaten 4 anhand der Ausgabedaten VAD. Somit wird das auftragskonforme Umpacken von Arzneimitteln durch einen doppelten Datenabgleich (Datencheck) gesichert, was für eine erhöhte Betriebssicherheit sorgt.

Bei einer Ausgestaltung des Arzneimittelumpacksystems 1 kann die Abgleichund Prüfstation 5 zum Durchführen eines visuellen Abgleichs ausgebildet sein, der insbesondere unter Nutzung einer Bild- oder Mustererkennung vorgenommen wird und in dessen Verlauf die Abgleich- und Prüfstation 5 entsprechende Visualisierungsdaten oder -datensätze VD erzeugt, die im Wesentlichen Bilddaten der untersuchten Verpackung 15 enthalten, ggf. bereits zusammen mit entsprechenden Erkennungsdaten, welche eine Identifizierung der in der Verpackung enthaltenen bzw. erkannten Arzneimitteleinheiten 14.1b, 14.2b ermöglichen. Wie bereits erwähnt, kann die Bilderkennung und das Erzeugen von Identifizierungsdaten auch durch die Ablaufsteuerungseinheit 6 übernommen werden, welche entsprechende Daten dem von der Abgleich- und Prüfstation erhaltenen Visualisierungsdatensatz VD hinzufügt.

So kann insbesondere die Abgleich- und Prüfstation 5 durch Vergleich mit bekannten Bild- oder Musterdaten, welche beispielsweise in der Speichereinheit 6a der Ablaufsteuerungseinheit 6 gespeichert sein können, in der Lage sein festzustellen, welche Art und Menge von Arzneimitteleinheiten in der patientenspezifischen Verpackung 15 enthalten sind. Wenn zusätzlich noch weitere Bestandteile des Patientenmedikamentationsdatensatzes auf der Verpackung 15 angegeben sind, beispielsweise durch Aufdrucken eines Barcodes oder entsprechenden Text, kann die Abgleich- und Prüfstation 5 bei geeigneter Ausges-taltung auch diese Daten erfassen und zur Erzeugung des Steuersignals SS5 auswerten. Zu diesem Zweck wäre in der Abgleich- und Prüfstation 5 eine entsprechende Auswerteeinheit vorzusehen, die in Figur 1 nicht explizit dargestellt ist.

Alternativ ist - wie bereits ausgeführt - eine Ausgestaltung möglich, bei der die Abgleich- und Prüfstation 5 lediglich die von ihr ermittelten Visualisierungsdaten VD bzw. Eigenschaften der Verpackung 15 an die Ablaufsteuerungseinheit 6 überträgt, welche dann ihrerseits den Vergleich mit den zugrunde liegenden Patientenmedikationsdaten PMD vornimmt und insbesondere bei Nichtkonsistenz ein entsprechendes Steuersignal (entsprechend dem Steuersignal SS5; in Figur 1 nicht gezeigt) auslöst. Gleiches gilt entsprechend für den Dosier- und Verpackungsautomaten 4 und die Ausgabedaten VAD.

Zu den angesprochenen Nichtkonsistenzen kann es insbesondere dann kommen, wenn im Betrieb des Dosier- und Verpackungsautomaten 4 ausgegebene Arzneimitteleinheiten nicht korrekt in die Verpackung 15 gelangen oder wenn die Behälter 4.1, 4.2, ..., 4.n fehlerhafte oder beschädigte Arzneimitteleinheiten enthalten. Möglich ist - wie gesagt - auch, dass der Automat 4 fälschlicherweise Verpackungen 15 erzeugt, die aktuell nicht mehr benötigt werden.

Derartige Fehler werden durch die Abgleich- und Prüfstation 5 bzw. den Dosier- und Verpackungsautomaten 4 erkannt und entsprechend der Ablaufsteuerungseinheit 6 mitgeteilt.

Insbesondere die korrekt hergestellten patientenspezifischen Verpackungen 15 verlassen die Abgleich- und Prüfstation 5 am rechten Abbildungsrand in Figur 1 vorzugsweise in Form eines Strangs 15' aus abtrennbar miteinander verbundenen, einzelnen patientenspezifischen Verpackungen 15, der bzw. die anschließend an den Auftraggeber (in der Regel den Benutzer des Rechnersystems 9; siehe oben) ausgeliefert werden können.

Wie aus der vorstehenden Beschreibung deutlich wurde, ist vordringlich die Ablaufsteuerungseinheit 6 für die Steuerung des auf dem System 1 ablaufenden Arzneimittelumpackverfahrens verantwortlich. Zu diesem Zweck führt sie geeignete Programmanweisungen aus, die auf dem Datenträgermedium 11 gespeichert sein können. Allerdings ist die vorliegende Erfindung keinesfalls auf die in Figur 1 lediglich exemplarisch dargestellte Datenübertragungs- und Steuerungsstruktur beschränkt. Insbesondere können bestimmte Funktionen der Ablaufsteuerungseinheit 6 auch dezentral an den betreffenden Stationen 2 - 5 des Arzneimittelumpacksystems 1 implementiert sein, wie dies insbesondere hinsichtlich der Abfüll- und Verpackungsstation 4 (Steuereinheit 4a) und der Abgleich- und Prüfstation 5 weiter oben bereits angeklungen war.

Des Weiteren können auch Funktionen der Ablaufsteuerungseinheit 6 und des optionalen Rechnersystems 8 in einem einzigen Rechner-/Steuerungssystem integriert sein.

Darüber hinaus können im Zuge anderer Ausgestaltungen des erfindungsgemäßen Arzneimittelumpacksystems 1 bestimmte Funktionen des zusätzlichen Rechnersystems 9, insbesondere Funktionen zum Erzeugen der Patientenmedikationsdatensätze PMD, auch als so genannte web-based (internetbasierte) Anwendungen ausgebildet sein, die auf einem Serversystem des Arzneimittelumpacksystems ablaufen und auf die ein Benutzer über das Internet 10 mittels eines externen Terminals zugreift, auf dem entsprechend außer einem WebBrowser keine zusätzliche Anwendungssoftware installiert sein muss.

Bei dem Ausführungsbeispiel gemäß Figur 1 ist die Ablaufsteuerungseinheit 6 dazu ausgebildet, sämtliche während des Verfahrensablaufs anfallende Datensätze, das heißt die arzneimittelspezifischen Datensätze ASD, die Patientenmedikationsdatensätze PMD, die verpackungsspezifischen Ausgabedaten VA-Dund die Visualisierungsdatensätze VD sowie die diesbezüglich erzeugten Steuersignale SS1-SS5' mit entsprechenden Verknüpfungen untereinander zu Dokumentationszwecken zu archivieren, insbesondere durch Speichern in der Speichereinheit 6a. Auf diese Weise lässt sich erfindungsgemäß der gesamte Umpackvorgang vom Entnehmen eines Arzneimittels 14.1, 14.2, ..., 14.n aus der entsprechenden ersten Verpackung 14.1a, 14.2a, ..., 14.na bis hin zur fertigen patientenspezifischen Verpackung 15 nachvollziehen, um auf diese Weise mögliche Fehlerquellen zu ermitteln und auszuschalten bzw. um Anforderungen im Rahmen des so genannten "good manufacturing practice" (GMP) gerecht zu werden, was insbesondere auf dem rechtlich hochsensiblen Arzneimittelsektor von großer Bedeutung ist.

Auch zu dem vorstehend genannten Zweck führt die Ablaufsteuerungseinheit 6 geeignete Programmanweisungen aus, die auf dem Datenträgermedium 11 gespeichert sind. Optional können die Dokumentations- und Archivierungsdaten auch dem weiteren Rechnersystem 8 zu Dokumentationszwecken zur Verfügung gestellt werden.

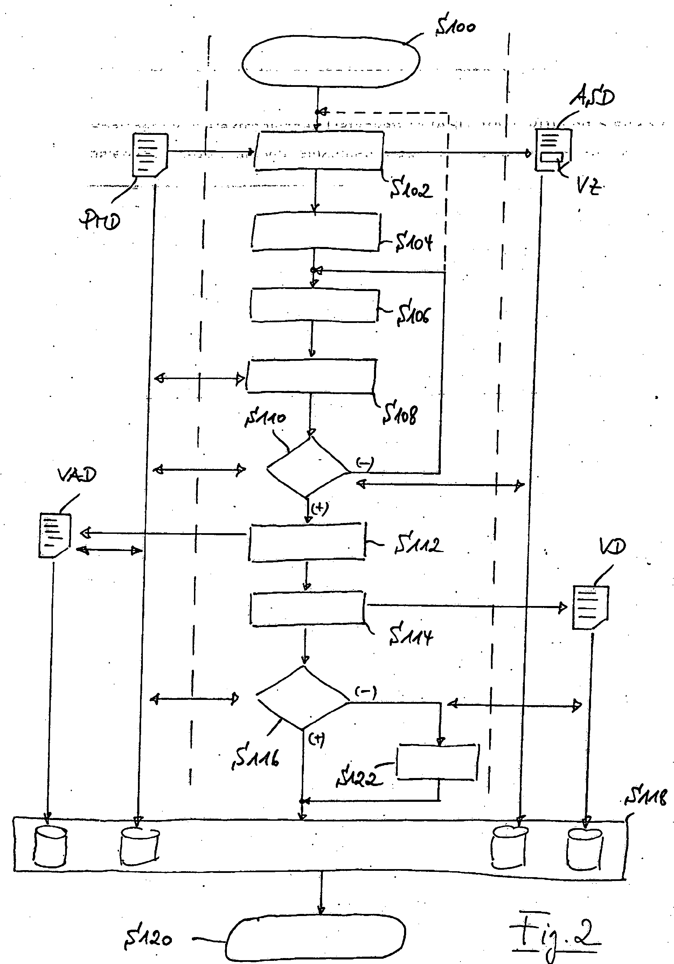

Figur 2 zeigt ein Flussdiagramm einer Ausgestaltung des erfindungsgemäßen Verfahrens zum Umpacken von Arzneimitteln. Der durchgängige, vertikale Ablaufstrang zwischen den vertikalen gestrichelten Linien in Figur 2 zeigt den eigentlichen Verfahrensverlauf, während links und rechts davon die Erzeugung bzw. die zeitliche Entwicklung der bereits anhand von Figur 1 erläuterten Datensätze PMD, ASD, VAD, VD und die darauf erfolgenden Zugriffe dargestellt ist.

Das Verfahren beginnt in Schritt S100. Daran schließt sich in Schritt S102 das Entpacken der Arzneimittel zum Erzeugen von Arzneimitteleinheiten an, beispielsweise durch Entblistern. Wie die Figur 2 im Zusammenhang mit Schritt S102 weiterhin zeigt, erfolgt das Entpacken in Schritt S102 nach Maßgabe von Patientenmedikationsdaten PMD (in Figur 2 links von Schritt S102 dargestellt) und bewirkt ein Erzeugen von arzneimittelspezifischen Daten ASD (in Figur 2 rechts von Schritt S102 dargestellt) welche eine Verwendbarkeitszeit VZ für die in Schritt S102 entnommenen Arzneimitteleinheiten beinhalten.

Der anschließende Schritt S104 symbolisiert die optionale Bevorratungslagerung der in Schritt S102 entnommenen Arzneimitteleinheiten. Anschließend erfolgt in Schritt S106 die Übergabe der Arzneimitteleinheiten in den Dosierund Verpackungsautomaten, wobei ein Abgleich mit den arzneimittelspezifischen Daten ASD erfolgt, was in Figur 2 durch einen Doppelpfeil rechts von Schritt S106 symbolisiert ist. Durch diesen Abgleich wird sichergestellt, dass die Ablaufsteuerungseinheit des Arzneimittelumpacksystems Informationen darüber enthält, an welchem Ort in dem Dosier- und Verpackungsautomaten ein bestimmtes Arzneimittel bzw. die entsprechenden Arzneimitteleinheiten enthalten ist bzw. sind.

In einem anschließenden Schritt S108 übermittelt die Ablaufsteuerungseinheit einen Auftrag zum Erzeugen patientenspezifischer Verpackungen an den Dosier- und Verpackungsautomaten. Zu diesem Zweck wird auf die Patientenmedikationsdaten PMD zugegriffen (Doppelpfeil links von Schritt S108). In einem anschließenden Schritt S110 wird überprüft, ob die zum Herstellen der gewünschten patientenspezifischen Verpackung erforderlichen Arzneimitteleinheiten in dem Dosier- und Verpackungsautomaten enthalten sind, das heißt ob überhaupt Einheiten eines gewünschten Arzneimittels in dem Automaten enthalten sind oder - optional - ob eine ausreichende Anzahl von Einheiten des gewünschten Arzneimittels vorhanden sind und ob diese eine Verwendbarkeitszeit VZ besitzen, die mit den zeitlichen Anforderungen der Patientenmedikationsdaten PMD kompatibel ist. Letzteres kann alternativ auch schon unmittelbar bei Auftragseingang (Erhalt der Patientenmedikationsdaten PMD) geprüft werden. Dabei erfordert Schritt S110 in dem ersten Fall einen Abgleich der Patientenmedikationsdaten PMD und der arzneimittelspezifischen Daten ASD, welche die Verwendbarkeitszeit VZ beinhalten. Dieser Abgleich ist in Figur 2 durch linke und rechte Doppelpfeile auf Höhe von Schritt S110 symbolisiert. Er wäre im Zuge der zweiten Alternative entsprechend früher auszuführen.

Fällt der Abgleich in Schritt S110 positiv aus (+), so wird anschließend in Schritt S112 die gewünschte patientenspezifische Verpackung erzeugt. Zudem werden in Schritt S112 auch die verpackungsspezifischen Ausgabedaten VAD erzeugt (in Figur 2 links außen dargestellt) und können anschließend mit dem Datensatz PMD verglichen werden, welcher die Auftragsdaten in ihrer aktuellen Version darstellt (vgl. den Doppelpfeil VAD - PMD in Figur 2). Dieser Abgleich ist in Figur 2 nicht explizit dargestellt, wurde jedoch weiter oben unter Bezugnahme auf die Figur 1 bereits ausführlich erläutert. Zur normalen Fortführung des Verfahrens müssen die die verglichenen Datensätze konsistent sein - anderenfalls wird ein entsprechendes Steuersignal ausgelöst.

In Schritt S114 wird die Verpackung zur abschließenden Überprüfung weitergeleitet. Fällt dagegen bereits der Abgleich in Schritt S110 negativ aus (-), so wird das Verfahren oberhalb von Schritt S106 fortgesetzt, falls noch geeignete Arzneimitteleinheiten in der Bevorratungslagerung verfügbar sind, oder das Verfahren kehrt nach Schritt S102 zurück, falls zum Erzeugen der gewünschten patientenspezifischen Verpackung zunächst neue Arzneimitteleinheiten entpackt werden müssen.

Im weiteren Verfahrensverlauf schließt sich an den bereits erwähnten Schritt S114, durch welchen weiterhin ein Erzeugen von Visualisierungsdaten VD (rechts in Figur 2) bewirkt ist, in Schritt S116 ein Abgleich der Visualisierungsdaten VD mit den Patientenmedikationsdaten PMD an. Optional kann der weiter oben angesprochene Abgleich der Ausgabedaten VAD der Automaten mit den Patientenmedikations- bzw. Auftragsdaten PMD auch im Wesentlichen simultan mit dem weiteren Abgleich in Schritt S116 erfolgen. Ist das Ergebnis des Abgleichs bzw. der Abgleiche in Schritt S116 positiv (+), d. h. die hergestellten patientenspezifischen Verpackungen entsprechen dem zugrunde liegenden aktuellen Patientenmedikationsdatensatz PMD, werden in Schritt S118 die Patientenmedikationsdaten PMD, die arzneimittelspezifischen Daten ASD, die Automaten-Ausgabedaten VAD und die Visualisierungsdaten VD sowie weitere, während des Verfahrensablaufs erzeugte Steuersignale, die in Figur 2 nicht explizit dargestellt sind, zu Dokumentationszwecken gespeichert, und das Verfahren endet in Schritt S120.

Fällt der Abgleich bzw. fallen die Abgleiche in Schritt S116 negativ aus (-), wird in Schritt S122 jeweils ein entsprechendes Steuersignal ausgelöst, und das Verfahren wird mit Schritt S118 fortgesetzt, wie oben beschrieben, wobei die Archivierung in Schritt S118 entsprechend auch das bzw. die in Schritt S122 ausgelöste(n) Steuersignal(e) mit umfasst.

Es sei an dieser Stelle ausdrücklich betont, dass grundsätzlich auch entweder nur der (erste) Abgleich der Datensätze VAD und PMD nach dem Ausgeben und Verpacken der Arzneimitteleinheiten, nur der (zweite) Abgleich der Datensätze VD und PMD nach der vorzugsweise visuellen Überprüfung der erzeugten Verpackungen oder in höchst vorteilhafter Weise beide Abgleiche durchgeführt werden können, um auf diese Weise durch doppelten Datencheck eine größtmögliche Betriebssicherheit zu erreichen.

Auf diese Weise ermöglicht die vorliegende Erfindung ein hochgradig automatisiertes Umpacken von Arzneimitteln zur Herstellung von patientenspezifischen Verpackungen, welches insbesondere mit Blick auf die Dokumentation und Nachvollziehbarkeit den hohen Anforderungen im Rahmen von GMP mit der Möglichkeit einer gewerblichen Nutzung gerecht wird.

## Patentansprüche

1. Verfahren zum Umpacken von Arzneimitteln (14.1, 14.2, ..., 14.n), wobei eine Anzahl von Arzneimitteleinheiten (14.1b, 14.2b, ..., 14.nb) in Form einer jeweiligen einzeldosierten Arzneiform eines entsprechenden Arzneimittels aus einer betreffenden ersten Verpackung (14.1a, 14.2a, ..., 14.na) entnommen und ein zugehöriger arzneimittelspezifischer Datensatz (ASD) erfasst wird und wobei wenigstens eine Teilmenge der entnommenen Arzneimitteleinheiten (14.1b, 14.2b, ..., 14.nb) an einen Dosier- und Verpackungsautomaten (4) übergeben wird, der wenigstens eine Arzneimitteleinheit (14.1b, 14.2b) aus einer Anzahl vorgegebener Arzneimittel (14.1, 14.2) entsprechend einem patientenspezifischen Medikationsdatensatz (PMD) ausgibt und in einer patientenspezifischen Verpackung (15) in Form eines Blister- oder Folienbeutels einpackt und dabei verpackungsspezifische Ausgabedaten (VAD) erzeugt, welche den in dem Dosier- und Verpackungsautomaten (4) ablaufenden Ausgabe- und Verpackungsvorgang dokumentieren, worauf ein automatischer Abgleich der verpackungsspezifischen Ausgabedaten (VAD) des Dosier- und Verpackungsautomaten (4) mit dem patientenspezifischen Medikationsdatensatz (PMD) vorgenommen und bei Nichtkonsistenz ein entsprechendes Steuersignal (SS5') ausgelöst wird,
und worauf weiterhin ein Inhalt der patientenspezifischen Verpackung (15) an einer Abgleich- und Prüfstation (5) automatisch überprüft wird, indem durch die Abgleich- und Prüfstation (5) ein entsprechender Datensatz (VD) erzeugt wird, der im Wesentlichen Bilddaten der untersuchten Verpackung (15) enthält, und durch die Abgleich- und Prüfstation (5) oder durch eine Ablaufsteuerungseinheit (6) mit dem patientenspezifischen Mediaktionsdatensatz (PMD) mittels eines visuellen Abgleichs unter Nutzung einer Bild- oder Mustererkennung verglichen wird, und bei Nichtkonsistenz ein entsprechendes Steuersignal (SS5) ausgelöst wird.

2. Verfahren nach Anspruch 1,
**dadurch gekennzeichnet,**
**dass** in dem arzneimittelspezifischen Datensatz (ASD) wenigstens eines der folgenden Daten erfasst wird:
- Art des Arzneimittels (14.1, 14.2, ..., 14.n), insbesondere seine Pharmazentralnummer, PZN;
- Anzahl der entnommenen und/oder fehlerhaften Arzneimitteleinheiten (14.1b, 14.2b, ..., 14.nb);
- Haltbarkeitsangaben;
- Aktualisierungsstand der Packungsbeilage/Produktinformationen.

3. Verfahren nach Anspruch 1 oder 2,
**dadurch gekennzeichnet,**
**dass** jedem arzneimittelspezifischen Datensatz (ASD) automatisch eine Verwendbarkeitszeit (VZ) in Abhängigkeit von der Entnahmezeit zugewiesen wird.

4. Verfahren nach mindestens einem der Ansprüche 1 bis 3,
**dadurch gekennzeichnet,**
**dass** eine automatische, insbesondere rechnergestützte Steuereinheit (6) zur Ansteuerung des Dosier- und Verpackungsautomaten (4) auf den arzneimittelspezifischen Datensatz (ASD) zugreift und dass das Ausgeben von Arzneimitteleinheiten (14.1b, 14.2b, ..., 14.nb) in Abhängigkeit von Daten erfolgt, die in dem arzneimittelspezifischen Datensatz (ASD) enthalten sind.

5. Verfahren nach mindestens einem der Ansprüche 1 bis 4,
**dadurch gekennzeichnet,**
**dass** eine automatische, insbesondere rechnergestützte Steuereinheit (6) zur Ansteuerung des Dosier- und Verpackungsautomaten (4) auf den Patientenmedikationsdatensatz (PMD) zugreift und dass das Ausgeben von Arzneimitteleinheiten (14.1b, 14.2b, .... 14.nb) in Abhängigkeit von Daten erfolgt, die in dem Patientenmedikationsdatensatz (PMD) enthalten sind.

6. Verfahren nach mindestens einem der Ansprüche 1 bis 5,
**dadurch gekennzeichnet,**
**dass** für Arzneimittel (14.1, 14.2, ..., 14.n), die sich beim Erfassen des arzneimittelspezifischen Datensatzes (ASD) lediglich hinsichtlich einer Packungsgröße der ersten Verpackung (14.1 a, 14.2a, ..., 14.na) unterscheiden, automatisch eine übergeordnete, gemeinsame Kennzeichnung zugewiesen wird, anhand der die entsprechenden Arzneimitteleinheiten (14.1b, 14.2b, ..., 14.nb) als ein gleiches Arzneimittel identifizierbar sind, insbesondere beim Ausgeben/Verpacken.

7. Verfahren nach mindestens einem der Ansprüche 1 bis 6,
**dadurch gekennzeichnet,**
**dass** bei den Verfahrensschritten der Entnahme der Arzneimitteleinheiten (14.1b, 14.2b, ..., 14.nb) aus der ersten Verpackung (14.1a, 14.2a, ..., 14.na), der Übergabe an den Dosier- und Verpackungsautomaten (4), dem Ausgeben/Verpacken der Arzneimitteleinheiten und/oder beim Abgleichen jeweils ein entsprechender Datensatz (ASD, VAD, VD) bzw. entsprechende Steuersignale (SS1-SS5'), insbesondere in elektronischer Form, erzeugt und archiviert und/oder dokumentiert wird bzw. werden, insbesondere durch Speichern in einer geeigneten elektronischen Speichereinheit (6a).

8. Verfahren nach mindestens einem der Ansprüche 1 bis 7,
**dadurch gekennzeichnet,**
**dass** die entnommenen Arzneimitteleinheiten (14.1b, 14.2b, ..., 14.nb) vor dem Übergeben an den Dosier- und Verpackungsautomaten (4) zwischengelagert und erst bei Bedarf an den Dosier- und Verpackungsautomaten (4) übergeben werden.

9. Arzneimittelumpacksystem (1), insbesondere zum Durchführen des Verfahrens nach mindestens einem der Ansprüche 1 bis 8, aufweisend:
- eine Entnahme- und Erfassungsstation (2), die zum Entnehmen einer Anzahl von Arzneimitteleinheiten (14.1b, 14.2b, ..., 14.nb) in Form jeweils einzeldosierter Arzneiformen eines entsprechenden Arzneimittels (14.1, 14.2, ..., 14.n) aus einer betreffenden ersten Verpackung (14.1a, 14.2a, ..., 14.na) und zum Erfassen eines zugehörigen arzneimittelspezifischen Datensatzes (ASD) in vorzugsweise elektronischer Form ausgebildet ist;
- optional eine Bevorratungsstation (3) zum Lagern der entnommenen Arzneimitteleinheiten getrennt nach Arzneimitteln;
- eine Abfüll- und Verpackungsstation mit einem Dosier- und Verpackungsautomaten (4), die zum Ausgeben wenigstens einer Arzneimitteleinheit aus einer Anzahl vorgegebener Arzneimittel entsprechend einem patientenspezifischen Medikationsdatensatz (PMD) und zum Einpacken derselben in eine patientenspezifische Verpackung (15) in Form eines Blister- oder Folienbeutels ausgebildet ist;
- eine Abgleich- und Prüfstation (5), die zum Überprüfen der patientenspezifischen Verpackung (15), insbesondere deren Inhalts, ausgebildet ist,
wobei die Abfüll- und Verpackungsstation zum Veranlassen eines ersten Abgleichs von verpackungsspezifischen Ausgabedaten (VAD), die von dem Dosier- und Verpackungsautomaten (4) beim Ausgeben und Verpacken erzeugt werden, mit dem patientenspezifischen Medikationsdatensatz (PMD) und zum Veranlassen des Ausgebens eines entsprechenden Steuersignals (SS5') bei Nichtkonsistenz ausgebildet ist und
wobei die Abgleich- und Prüfstation (5) zum Veranlassen eines zweiten Abgleichens der patientenspezifischen Verpackung (15), insbesondere deren Inhalts, mit dem patientenspezifischen Medikationsdatensatz (PMD) und zum Veranlassen des Auslösens eines entsprechenden Steuersignals (SS5) bei Nichtkonsistenz ausgebildet ist, wobei die Abgleich- und Prüfstation (5) dazu ausgebildet ist, einen Visualisierungsdatensatz (VD) zu erzeugen, der im Wesentlichen Bilddaten der untersuchten Verpackung (15) enthält, und wobei die Abgleich- und Prüfstation (5) oder eine Ablaufsteuerungseinheit (6) des Arzneimittelumpacksystems (1) dazu ausgebildet ist, den zweiten Abgleich mittels eines visuellen Abgleichs unter Nutzung einer Bild- oder Mustererkennung vorzunehmen,
wobei die Entnahme- und Erfassungsstation (2) mit der Abfüll- und Verpackungsstation (4) und mit der Abgleich- und Prüfstation (5) daten- und steuerungstechnisch verbunden ist, um den ersten und den zweiten Abgleich vorzunehmen, und wobei zumindest die Entnahme- und Erfassungsstation (2), die Abfüll- und Verpackungsstation (4) und die Abgleich- und Prüfstation (5) mit der übergeordneten, insbesondere rechnergesteuerten Ablaufsteuerungseinheit (6) daten- und steuerungstechnisch verbunden sind.

10. System nach Anspruch 9,
**gekennzeichnet durch**
eine elektronische Datenspeichereinheit (6a), insbesondere in daten- und signaltechnischer Verbindung mit der Ablaufsteuerungseinheit (6), zum Speichern von elektronischen Datensätzen (ASD, VAD, VD) und Steuersignalen (SS1-SS5'), die von einzelnen Stationen (2-5) gemäß Anspruch 9 erzeugt werden, insbesondere zum Zwecke einer Dokumentation/Archivierung gemäß Anspruch 7.

11. Computerprogrammprodukt, mit auf einem Datenträger (11) gespeicherten ersten Programmanweisungen, die zur Ausführung durch die Ablaufsteuerungseinheit (6) gemäß Anspruch 9 vorgesehen sind, um die elektronischen Datensätze (ASD, VAD, VD) und die Steuersignale (SS1-SS5') gemäß Anspruch 7 zu erzeugen und/oder zu archivieren und/oder zu visualisieren.

12. Computerprogrammprodukt nach Anspruch 11,
**gekennzeichnet durch** zweite Programmanweisungen zum Verarbeiten der während des Verfahrens gemäß mindestens einem der Ansprüche 1 bis 8 erzeugten Steuersignale (SS1-SS5'), um das Verfahren bei Nichtkonsistenz zu unterbrechen und/oder das Eingreifen einer Bedienperson zu veranlassen.

## Claims

1. Method of repackaging medicaments (14.1, 14.2, ..., 14.n), wherein a number of medicament units (14.1b, 14.2b, ..., 14.nb), each in the form of a single-dose dosage form of a corresponding medicament, is removed from a respective first packaging (14.1a, 14.2a, ..., 14.na) and an associated medicament-specific dataset (ASD) is acquired and wherein at least some of the medicament units removed (14.1b, 14.2b, ..., 14.nb) are transferred to a dispensing and packaging machine (4) which dispenses at least one medicament unit (14.1b, 14.2b) from a number of specified medicaments (14.1, 14.2) in accordance with a patient-specific medication dataset (PMD) and packages it in a patient-specific packaging (15) in the form of a blister pack or foil pouch and at the same time generates packaging-specific dispensing data (VAD) which record the dispensing and packaging operation taking place in the dispensing and packaging machine (4), whereupon an automatic comparison of the packaging-specific dispensing data (VAD) of the dispensing and packaging machine (4) with the patient-specific medication dataset (PMD) is carried out and in the event of inconsistency a corresponding control signal (SS5') is triggered, and whereupon, furthermore, a content of a patient-specific packaging (15) is automatically checked at a comparison and checking station (5), in which check a corresponding dataset (VD) is generated by the comparison and checking station (5), which dataset contains substantially image data of the packaging (15) being examined, and is compared with the patient-specific medication dataset (PMD) by the comparison and checking station (5) or by a process control unit (6) by means of a visual comparison using image-recognition or pattern-recognition, and in the event of inconsistency a corresponding control signal (SS5) is triggered.

2. Method according to claim 1,
**characterised in that**
in the medicament-specific dataset (ASD) at least one of the following data is acquired:
- type of medicament (14.1, 14.2, ..., 14.n), especially its pharmaceutical central number, PZN;
- number of removed and/or defective medicament units (14.1b, 14.2b, ..., 14.nb);
- shelf-life information;
- update status of the package insert/product information.

3. Method according to claim 1 or 2,
**characterised in that**
each medicament-specific dataset (ASD) is automatically allocated a usability period (VZ) in dependence upon the removal time.

4. Method according to at least one of claims 1 to 3, **characterised in that**
an automatic, especially computer-assisted control unit (6) for controlling the dispensing and packaging machine (4) accesses the medicament-specific dataset (ASD), and the dispensing of medicament units (14.1b, 14.2b, ..., 14.nb) is effected in dependence upon data contained in the medicament-specific dataset (ASD).

5. Method according to at least one of claims 1 to 4,
**characterised in that**
an automatic, especially computer-assisted control unit (6) for controlling the dispensing and packaging machine (4) accesses the patient medication dataset (PMD), and the dispensing of medicament units (14.1b, 14.2b, ..., 14.nb) is effected in dependence upon data contained in the patient medication dataset (PMD).

6. Method according to at least one of claims 1 to 5,
**characterised in that**
for medicaments (14.1, 14.2, ..., 14.n) which, on acquisition of the medicament-specific dataset (ASD), differ only in respect of the pack size of the first packaging (14.1a, 14.2a, ..., 14.na) there is automatically allocated a higher-level, common identifier with reference to which the corresponding medicament units (14.1b, 14.2b, ..., 14.nb) are identifiable as being an identical medicament, especially during dispensing/packaging.

7. Method according to at least one of claims 1 to 6,
**characterised in that**
during the method steps of the removal of the medicament units (14.1b, 14.2b, ..., 14.nb) from the first packaging (14.1a, 14.2a, ..., 14.na), the transfer to the dispensing and packaging machine (4) and the dispensing/packaging of the medicament units and/or during the comparison, in each case a corresponding dataset (ASD, VAD, VD) or corresponding control signals (SS1-SS5'), especially in electronic form, is/are generated and archived and/or recorded, especially by storage in a suitable electronic storage unit (6a).

8. Method according to at least one of claims 1 to 7,
**characterised in that**
the removed medicament units (14.1b, 14.2b, ..., 14.nb) are stored intermediately prior to transfer to the dispensing and packaging machine (4) and are transferred to the dispensing and packaging machine (4) only as required.

9. Medicament repackaging system (1), especially for carrying out the method according to at least one of claims 1 to 8, having:
- a removal and acquisition station (2), which is configured for removing a number of medicament units (14.1b, 14.2b, ..., 14.nb), in the form of single-dose dosage forms of a corresponding medicament (14.1, 14.2, ..., 14.n), from a respective first packaging (14.1a, 14.2a, ..., 14.na) and for acquiring an associated medicament-specific dataset (ASD) preferably in electronic form;
- optionally a storage station (3) for storing the removed medicament units separately according to the medicaments;
- a filling and packaging station with a dispensing and packaging machine (4) which is configured for dispensing at least one medicament unit from a number of specified medicaments in accordance with a patient-specific medication dataset (PMD) and for packaging of that medicament unit in a patient-specific packaging (15) in the form of a blister pack or foil pouch;
- a comparison and testing station (5) which is configured for checking the patient-specific packaging (15), especially the contents thereof,
wherein the filling and packaging station is configured for prompting a first comparison of packaging-specific dispensing data (VAD), which are generated by the dispensing and packaging machine (4) during dispensing and packaging, with the patient-specific medication dataset (PMD) and for prompting the output of a corresponding control signal (SS5') in the event of inconsistency and wherein the comparison and checking station (5) is configured for prompting a second comparison of the patient-specific packaging (15), especially the contents thereof, with the patient-specific medication dataset (PMD) and for prompting the triggering of a corresponding control signal (SS5) in the event of inconsistency, the comparison and checking station (5) being configured for generating a visualisation dataset (VD) which contains substantially image data of the packaging (15) being examined, and the comparison and checking station (5) or a process control unit (6) of the medicament repackaging system (1) being configured for carrying out the second comparison by means of a visual comparison using image-recognition or pattern-recognition,
wherein the removal and acquisition station (2) is in data and control communication with the filling and packaging station (4) and with the comparison and checking station (5) in order to carry out the first and second comparisons, and wherein at least the removal and acquisition station (2), the filling and packaging station (4) and the comparison and checking station (5) are in data and control communication with the higher-level, especially computer-controlled process control unit (6).

10. System according to claim 9,
**characterised by**
an electronic data storage unit (6a), especially in data and signal communication with the process control unit (6), for storing electronic datasets (ASD, VAD, VD) and control signals (SS1-SS5') which are generated by individual stations (2-5) according to claim 9, especially for the purpose of recording/archiving according to claim 7.

11. Computer program product, having first program instructions stored on a data storage medium (11), which instructions are provided so as to be carried out by the process control unit (6) according to claim 9, in order to generate and/or archive and/or visualise the electronic datasets (ASD, VAD, VD) and the control signals (SS1-SS5') according to claim 7.

12. Computer program product according to claim 11,
**characterised by** second program instructions for processing the control signals (SS1-SS5') generated during the method according to at least one of claims 1 to 8 in order to interrupt the method and/or to prompt the intervention of an operator in the event of inconsistency.

## Revendications

1. Procédé pour reconditionner des médicaments (14.1, 14.2, ..., 14.n), sachant qu'un certain nombre d'unités médicamenteuses (14.1b, 14.2b, ..., 14.nb) sous la forme d'une forme médicamenteuse respective monodose d'un médicament correspondant est prélevé d'un premier conditionnement correspondant (14.1a, 14.2a, ..., 14.na) et qu'un jeu de données associé (ASD) spécifique du médicament est collecté, et sachant qu'au moins une quantité partielle des unités médicamenteuses prélevées (14.1b, 14.2b, ..., 14.nb) est transférée à un automate (4) de dosage et de conditionnement, qui délivre au moins une unité médicamenteuse (14.1b, 14.2b) constituée d'un nombre de médicaments prédéfinis (14.1, 14.2) conformément à un jeu (PMD) de données de médication spécifique d'un patient, et les emballe dans un conditionnement (15) spécifique du patient sous la forme d'un blister ou d'un sachet, en produisant alors des données de délivrance (VAD) spécifiques du conditionnement, qui documentent le processus de délivrance et de conditionnement se déroulant dans l'automate (4) de dosage et de conditionnement, à la suite de quoi une comparaison automatique des données de délivrance (VAD) spécifiques du conditionnement de l'automate (4) de dosage et de conditionnement avec le jeu (PMD) de données de médication spécifique du patient est entreprise et un signal de commande correspondant (SS5') est déclenché en cas d'absence de cohérence, et à la suite de quoi en outre le contenu du conditionnement (15) spécifique du patient est vérifié à un poste (5) de comparaison et de contrôle, par le fait qu'un jeu de données correspondant (VD), qui contient pour l'essentiel des données image du conditionnement analysé (15), est produit par le poste (5) de comparaison et de contrôle, et est comparé par le poste (5) de comparaison et de contrôle ou par une unité (6) de commande séquentielle avec le jeu (PMD) de données de médication spécifique du patient au moyen d'une comparaison visuelle en utilisant une reconnaissance d'image ou de forme, et qu'un signal de commande correspondant (SS5) est déclenché en cas d'absence de cohérence.

2. Procédé selon la revendication 1, **caractérisé en ce qu'**au moins une des données suivantes est collectée dans le jeu de données (ASD) spécifique du médicament :
- type du médicament (14.1, 14.2, ..., 14.n), en particulier son numéro de référence (abréviation allemande PNZ - Pharmazentralnummer) ;
- nombre d'unités médicamenteuses prélevées et/ou défectueuses (14.1b, 14.2b, ..., 14.nb) ;
- indications de durée de conservation ;
- état d'actualisation de la notice de conditionnement/des informations produit.

3. Procédé selon la revendication 1 ou 2, **caractérisé en ce qu'**une durée d'utilisation (VZ) est automatiquement affectée à chaque jeu de données (ASD) spécifique du médicament en fonction de l'instant de prélèvement.

4. Procédé selon au moins une des revendications 1 à 3, **caractérisé en ce qu'**une unité de commande (6) automatique, en particulier assistée par ordinateur, accède au jeu de données (ASD) spécifique du médicament afin de commander l'automate (4) de dosage et de conditionnement, et **en ce que** la délivrance d'unités médicamenteuses (14.1b, 14.2b, ..., 14.nb) s'effectue en fonction des données qui sont contenues dans le jeu de données (ASD) spécifique du médicament.

5. Procédé selon au moins une des revendications 1 à 4, **caractérisé en ce qu'**une unité de commande (6) automatique, en particulier assistée par ordinateur, accède au jeu (PMD) de données de médication du patient afin de commander l'automate (4) de dosage et de conditionnement, et **en ce que** la délivrance d'unités médicamenteuses (14.1b, 14.2b, ..., 14.nb) s'effectue en fonction des données qui sont contenues dans le jeu (PMD) de données de médication du patient.

6. Procédé selon au moins une des revendications 1 à 5, **caractérisé en ce que**, pour des médicaments (14.1, 14.2, ..., 14.n) qui lors de la collecte du jeu de données (ASD) spécifique du médicament diffèrent uniquement quant à une taille d'emballage du premier conditionnement (14.1a, 14.2a, ..., 14.na), on affecte automatiquement une identification principale commune à l'aide de laquelle les unités médicamenteuses correspondantes (14.1b, 14.2b, ..., 14.nb) peuvent être identifiées comme un même médicament, en particulier lors de la délivrance/du conditionnement.

7. Procédé selon au moins une des revendications 1 à 6, **caractérisé en ce que**, lors des étapes de prélèvement des unités médicamenteuses (14.1b, 14.2b, ..., 14.nb) à partir du premier conditionnement (14.1a, 14.2a, ..., 14.na), de transfert à l'automate (4) de dosage et de conditionnement et de délivrance/conditionnement des unités médicamenteuses, et/ou lors de la comparaison, un jeu de données correspondant (ASD, VAD, VD) ou des signaux de commande correspondant (SS1-SS5') est ou sont respectivement produits et archivés et/ou documentés, en particulier sous une forme électronique, notamment par mémorisation dans une unité électronique (6a) de mémorisation appropriée.

8. Procédé selon au moins une des revendications 1 à 7, **caractérisé en ce que** les unités médicamenteuses prélevées (14.1b, 14.2b, ..., 14.nb) sont entreposées avant le transfert à l'automate (4) de dosage et de conditionnement, et ne sont transférées à l'automate (4) de dosage et de conditionnement qu'en cas de besoin.

9. Système (1) de reconditionnement de médicaments, en particulier pour la mise en oeuvre du procédé selon au moins une des revendications 1 à 8, présentant :
- un poste (2) de prélèvement et de collecte, qui est conçu pour prélever d'un premier conditionnement correspondant (14.1a, 14.2a, ..., 14.na) un certain nombre d'unités médicamenteuses (14.1b, 14.2b, ..., 14.nb) sous la forme de formes médicamenteuses respectives monodoses d'un médicament correspondant (14.1, 14.2, ..., 14.n), et pour collecter un jeu de données associé (ASD) spécifique du médicament, de préférence sous une forme électronique ;
- optionnellement, un poste de stockage (3) pour stocker les unités médicamenteuses prélevées, séparément par médicaments ;
- un poste de remplissage et de conditionnement avec un automate (4) de dosage et de conditionnement, qui est conçu pour délivrer au moins une unité médicamenteuse constituée d'un nombre de médicaments prédéfinis conformément à un jeu (PMD) de données de médication spécifique d'un patient, et pour les emballer dans un conditionnement (15) spécifique du patient sous la forme d'un blister ou d'un sachet ;
- un poste (5) de comparaison et de contrôle, qui est conçu pour vérifier le conditionnement (15) spécifique du patient, en particulier son contenu,
sachant que le poste de remplissage et de conditionnement est conçu pour provoquer une première comparaison de données de délivrance (VAD) spécifiques du conditionnement, qui sont produites par l'automate (4) de dosage et de conditionnement lors de la délivrance et du conditionnement, avec le jeu (PMD) de données de médication spécifique du patient, et pour provoquer la délivrance d'un signal de commande correspondant (SS5') en cas d'absence de cohérence,
et sachant que le poste (5) de comparaison et de contrôle est conçu pour provoquer une deuxième comparaison du conditionnement (15) spécifique du patient, en particulier de son contenu, avec le jeu (PMD) de données de médication spécifique du patient, et pour provoquer le déclenchement d'un signal de commande correspondant (SS5) en cas d'absence de cohérence, sachant que le poste (5) de comparaison et de contrôle est conçu pour produire un jeu (VD) de données de visualisation qui contient pour l'essentiel des données image du conditionnement analysé (15), et sachant que le poste (5) de comparaison et de contrôle ou une unité (6) de commande séquentielle du système (1) de reconditionnement de médicaments est conçu pour entreprendre la deuxième comparaison au moyen d'une comparaison visuelle en utilisant une reconnaissance d'image ou de forme,
sachant que le poste (2) de prélèvement et de collecte est relié en matière de transmission de données et de commande au poste (4) de remplissage et de conditionnement et au poste (5) de comparaison et de contrôle, afin d'entreprendre la première et la deuxième comparaison, et sachant qu'au moins le poste (2) de prélèvement et de collecte, le poste (4) de remplissage et de conditionnement et le poste (5) de comparaison et de contrôle sont reliés en matière de transmission de données et de commande à l'unité principale (6) de commande séquentielle, notamment pilotée par ordinateur.

10. Système selon la revendication 9, **caractérisé par** une unité électronique (6a) de mémorisation de données, en particulier reliée en matière de transmission de données et de commande à l'unité (6) de commande séquentielle, pour mémoriser des jeux de données électroniques (ASD, VAD, VD) et des signaux de commande (SS1-SS5') qui sont produits par les différents postes (2-5) selon la revendication 9, en particulier aux fins d'une documentation/d'un archivage selon la revendication 7.

11. Produit-programme informatique, avec des premières instructions de programme mémorisées sur un support de données (11), qui sont prévues pour être exécutées par l'unité (6) de commande séquentielle selon la revendication 9, afin de produire et/ou d'archiver et/ou de visualiser les jeux de données électroniques (ASD, VAD, VD) et les signaux de commande (SS1-SS5') selon la revendication 7.

12. Produit-programme informatique selon la revendication 11, **caractérisé par** des deuxièmes instructions de programme pour traiter les signaux de commande (SS1-SS5') produits pendant le procédé selon au moins une des revendications 1 à 8, afin d'interrompre le procédé en cas d'absence de cohérence et/ou de provoquer l'intervention d'un opérateur.
